(19)

Europäisches
Patentamt

European
Patent Office

Office européen
des brevets

(11) **EP 2 462 950 A1**

(12) **EUROPEAN PATENT APPLICATION**

(43) Date of publication:
**13.06.2012 Bulletin 2012/24**

(51) Int Cl.:
*A61K 39/39* (2006.01)          *A61K 47/48* (2006.01)
*C07K 14/525* (2006.01)

(21) Application number: **10194240.7**

(22) Date of filing: **08.12.2010**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Neovacs**
**75014 Paris (FR)**

(72) Inventors:
• **Grouard-Vogel, Géraldine**
**75014 Paris (FR)**

• **Dhellin, Olivier**
**75020 Paris (FR)**
• **Fanget, Bernard**
**42800 Châteauneuf (FR)**
• **Vandepapelière, Pierre**
**5021 Bonnine (BE)**

(74) Representative: **Icosa**
**83 avenue Denfert-Rochereau**
**75014 Paris (FR)**

Remarks:
Amended claims in accordance with Rule 137(2)
EPC.

(54) **Strongly inactivated and still highly immunogenic vaccine, and process of manufacturing thereof**

(57)    The present invention relates to an immunogenic product comprising TNFα coupled with KLH, wherein the TNFα is strongly inactivated, which means that the product shows less than 30% of cytolytic activity and/or an inactivation factor of more than 15000, in the conditions of TEST A; an emulsion and a vaccine comprising thereof and methods for preparing said immunogenic product.

EP 2 462 950 A1

**Description**

## FIELD OF THE INVENTION

This invention relates to the field of the prevention or treatment of diseases where an antibody response against endogenous TNF$\alpha$ is sought. This invention relates to novel immunogenic products that induce, when administered to a mammal host, an immune response with anti-TNF$\alpha$ antibody production in said mammal host.

## BACKGROUND OF THE INVENTION

Tumor necrosis factor alpha (TNF$\alpha$) consists of a homotrimeric, pleiotropic cytokine, and is secreted in response to inflammatory stimuli in diseases such as for example rheumatoid arthritis, inflammatory bowel disease and psoriasis.

The pathological activities of TNF$\alpha$ have attracted much attention. Although TNF$\alpha$ causes necrosis of some types of tumors, this cytokine promotes the growth of other types of tumor cells. In general, high levels of TNF$\alpha$ correlate with increased risk of mortality. TNF$\alpha$ participates in both inflammatory disorders of inflammatory and non-inflammatory origin. In sepsis, the release of high amounts of TNF$\alpha$ causes a major failure in a variety of body organs with a high risk of death. Abnormal TNF$\alpha$ production is encountered both in various chronic and acute diseases. High levels of endogenous production of TNF$\alpha$, even if TNF production is transient, is known to lead to shock and tissue injury, catabolic hormone release, vascular leakage syndrome, adult respiratory distress disorder, gastrointestinal necrosis, acute renal tube necrosis, adrenal haemorrhage, decreased muscle membrane potentials, disseminated intravascular coagulation and fever. Weak but chronic (over)production of TNF$\alpha$ is known to cause weight loss, anorexia, protein catabolism, lipid depletion, hepatosplenomegaly, subendocardial inflammation, insulin resistance, acute phase protein release and endothelial activation.

TNF$\alpha$ consists of a mediator substance in various diseases including septic shock, cancer, AIDS, transplantation rejection, multiple sclerosis, diabetes, rheumatoid arthritis, trauma, malaria, meningitis, ischemia-reperfusion injury and adult respiratory distress syndrome. This explains why a substantial amount of research has been conducted for designing anti-TNF$\alpha$ therapies.

One kind of anti-TNF$\alpha$ therapy, which may also be termed passive immunotherapy, involves the administration of anti-TNF$\alpha$ monoclonal antibodies to the patients in need thereof. Various anti-TNF$\alpha$ monoclonal antibodies are tested in clinical trials or are already actually used in medical treatment of anti-TNF$\alpha$-related diseases. It may be cited the following anti-TNF$\alpha$ monoclonal antibodies : Afelimomab (presently endowing clinical trials), Certolizumab (authorised for rheumatoid arthritis and Crohn's disease), Golimumab (authorised for rheumatoid arthritis, psoriatic arthritis and ankylosing spondylitis), Infliximab (authorised for rheumatoid arthritis, psoriatic arthritis, ankylosing spondylitis, plaque psoriasis, Crohn's disease and ulcerative colitis), and Adalimumab (authorised for rheumatoid arthritis, juvenile idiopathic arthritis, ankylosing spondylitis, plaque psoriasis, psoriatic arthritis, Crohn's disease ).

The above cited anti-TNF$\alpha$ monoclonal antibodies have proved their therapeutic activity in TNF$\alpha$-related diseases. However, these monoclonal antibodies are endowed with the various known drawbacks of therapeutic antibodies in general, which includes the induction of an antibody response of the host against the monoclonal antibodies which leads rapidly to a decreasing efficacy of the therapeutic anti-TNF$\alpha$ monoclonal antibodies.

As an alternative medical anti-TNF$\alpha$ strategy to monoclonal antibodies, some authors have suggested to design active immunotherapy treatments based on the induction of anti-TNF$\alpha$ antibody production in the patients. Illustratively, vaccines containing modified TNF$\alpha$ molecules are described in the PCT Application WO 98/46642. The immunogenic compound described in this PCT Application consists of a modified TNF$\alpha$ protein where a portion of the native amino acid sequence has been replaced by one or more polypeptides bearing T cell epitopes. In some embodiments, the said modified TNF$\alpha$ molecules may be conjugated to an anti-Fc$\gamma$RI antibody fragment. WO02/11759 describes vaccines against cytokines, including the coupling of a cytokine, such as for example VEGF, with an activated carrier molecule, for example activated KLH. In this patent application, KLH is contacted with glutaraldehyde, and then added to a solution of VEGF. In the resulting product, the biological activity of the VEGF cytokine is not inactivated.

It was then understood that the cytokine biological activity had to be neutralized for two reasons. First, some cytokines, such as TNF$\alpha$, drive inflammation and organ alterations in their endogeneous state, and second, in the context of cytokine overproduction conditions, a vaccine should not be recognized in vivo as an additional source of cytokines.

PCT application WO 2004/024189 disclosed immunogenic products comprising molecular associations between (i) an antigenic protein of interest and (ii) a carrier protein, and wherein (i) and (ii) were partly bound together by covalent bonds and partly bound together by non-covalent bonds. In this PCT application, it was disclosed that the high number of antigenic molecules of interest associated with the carrier protein, mainly by non-covalent bonds, was a condition for a final product with a high immunogenicity.

PCT application WO 2007/022813 in the name of the Applicant disclosed an immunogenic product comprising heterocomplexes between TNF$\alpha$ molecules and KLH molecules, where TNF$\alpha$ inactivation had been improved as compared with the level of TNFα inactivation found for the corresponding immunogenic compounds disclosed in the PCT Application WO 2004/24189 discussed above. More precisely, the examples showed that optimal inactivation of the TNFα cytotoxic activity was reached when performing a step of chemical treatment of the pre-formed heterocomplexes with formaldehyde during a period of time ranging from 96 hours to 192 hours. Notably, it was specified that performing the formaldehyde treatment step for a period of time of more than 192 hours at a concentration of 66mM led to a final product that was highly stable but with a significantly lowered ability to induce antibodies having a high neutralizing activity against endogeneous TNFα. In fact, in this patent application, it was assessed that going further in inactivation would necessary lead to a significant loss of the antigenic/immunogenic properties of the resulting product.

The Applicant now believes that, even though the prior art products included inactivated cytokines, said inactivation was not fully optimized and that it is now possible to overcome the technical prejudice preventing the skilled artisan from further inactivating the cytokines in a cytokine-carrier protein vaccine.

The product of the invention is an immunogenic product comprising cytokines coupled with carrier proteins, in which cytokines have lost most of their biological activity but yet retain their natural immunogenicity. The product of the invention thus shows a high degree of safety, a strong inactivation treatment of the TNFα biological activity and still very good anti-TNFα immunogenic properties.

## SUMMARY OF THE INVENTION

Consequently, one object of the invention includes an immunogenic product comprising TNFα coupled with KLH, wherein the TNFα is strongly inactivated, which means that the product shows less than 30% of cytolytic activity and/or an inactivation factor of more than 15000 in the conditions of hereunder cited TEST A; an emulsion comprising said product with combination to an oil and a surfactant; and a vaccine comprising said product or emulsion.

In this invention, the term "TNFα coupled with KLH" means that covalent and/or non-covalent bounds link TNFα to KLH.

According to an embodiment, the product of the invention may comprise free TNFα homopolymers; preferentially the percentage of free TNFα homopolymers of more than 300kDa is of less than 30% w/w of total TNFα. Preferably, the percentage of free TNFα homopolymers is calculated according to Test C.

This invention goes even further in inactivation, and ensures that the vaccine of the invention, in the conditions of temperature of the human body, i.e. *in vivo* temperature conditions, typically at 37°C, will remain inactive during the necessary time, i.e. the time during which the immunization has to be effective. In this regard, Test B was designed, in conformity with the European and American Pharmacopeia. In the meaning of this invention, the terms "remain inactive" or "inactive overtime", mean that the product shows less than 80% of cytolytic activity in the conditions of TEST B and/or has an inactivation factor of more than 500.

According to an embodiment and for storage purposes, the product or the vaccine composition of the invention may be lyophilized.

This invention also relates to a formulation of the product of the invention, wherein the product is within an emulsion. Such emulsion comprises the product of the invention, an oil and a surfactant or a mixture of at least one oil and at least one surfactant.

This invention also pertains to a vaccine composition comprising a product as described in the present specification, in combination with one or more immunoadjuvants. An immunoadjuvant may be any substance that enhances the immune response of the product or vaccine composition of the invention with which it is combined or mixed.

This invention also pertains to a kit comprising at least one vial containing the lyophilized product of the invention, at least one vial containing water for injection, and at least one vial containing adjuvant, and means for mixing the product and the water in order to obtain an aqueous solution, and for contacting said solution to the adjuvant, and for emulsifying the mixture of the aqueous solution with the adjuvant. According to an embodiment, said means are a syringe. The kit also includes at least one needle. Preferably, the kit includes two needles.

This invention also relates to the medical device comprising the product of the invention or the vaccine composition of the invention.

This invention also relates to a method for preparing a product comprising TNFα coupled with KLH, wherein the TNFα is strongly inactivated, which means that the product shows less than 30% of cytolytic activity in the conditions of TEST A, comprising the steps of:

a) mixing together (i) purified TNFα, (ii) purified Keyhole limpet hemocyanin and (iii) glutaraldehyde
b) removing compounds having a molecular weight of less than 10 kDa, or of less than 8 kDa
characterized in that, after step b), the following steps are performed:
c) adding formaldehyde in a concentration/time of reaction condition ranging from at least 60 mM for at least 10 days (240 hours) to at least 120 mM for at least 6 days (144 hours); in an embodiment, formaldehyde is applied in a concentration of at least 200 mM during at least 10 days (240 hours) ; in a preferred embodiment, formaldehyde is added in order to reach a concentration of 220 mM to 270 mM in the medium, during a period of time of more than 300 hours;

d) blocking the reaction with formaldehyde by adding a quenching compound selected from (i) a reducing agent and (ii) an amino acid selected from the group consisting of lysine and glycine and mixture thereof,

e) collecting the said immunogenic product.

Advantageously, in step a) glutaraldehyde is applied in a concentration/time of reaction condition of at least 20 mM for more than 120 minutes, preferably for more than 240 minutes. According to an embodiment, the reaction with glutaraldehyde (step a) is stopped prior to removing compounds having a molecular weight of less than 10 kDa, (step b) by adding a quenching compound, preferably a quenching compound that is selected from (i) a reducing agent and (ii) an amino acid selected from the group consisting of lysine and glycine and mixture thereof.

According to a preferred embodiment of the invention, just prior to collecting at step f), a step of tangential flow filtration using a filtration membrane having a cut-off value of at least 100 kDa (preferentially 300 kDa) is performed, resulting in that the substances having a molecular weight of less than 100 (preferably 300 kDa) are removed from the product.

In a variant of the invention, the method for preparing a product comprising TNFα coupled with KLH, wherein the TNFα is strongly inactivated, which means that the product shows less than 30% of cytolytic activity in the conditions of TEST A, comprises the steps of:

a) mixing together (i) purified TNFα, (ii) purified Keyhole limpet hemocyanin and (iii) glutaraldehyde

b) removing compounds having a molecular weight of less than 10 kDa and is characterized in that in step a) glutaraldehyde is applied at a concentration of at least 20mM, preferably 25 mM during more than 18 hours, the reaction with glutaraldehyde is stopped by adding a quenching compound, preferably a quenching compound that is selected from (i) a reducing agent and (ii) an amino acid selected from the group consisting of lysine and glycine and mixture thereof, and then the product is collected.

In an embodiment, after step b) and prior to collecting the product, formaldehyde is applied in a concentration/ time of reaction condition ranging from at least 60 to 240 mM/at least 4 days, and then the reaction with formaldehyde is blocked by adding a quenching compound selected from (i) a reducing agent and (ii) an amino acid selected from the group consisting of lysine and glycine and mixture thereof.

According to a preferred embodiment of the invention, just prior to collecting the product, a step of tangential flow filtration using a filtration membrane having a cut-off value of at least 100 kDa (preferentially 300 kDa) is performed, resulting in that the substances having a molecular weight of less than 100 kDa (preferably 300 kDa) are removed from the product.

The present invention also relates to a method for preparing an immunogenic product that is useful for inducing an anti-TNFα antibody response in a host to whom the said immunogenic product is administered. The produced immunogenic product is mainly used in vaccine compositions for preventing or treating a disease linked to an over-production of TNFα. More specifically, this invention relates to a method for preventing or treating a disease linked to an over-production of TNFα comprising a step of administering to the animal, including a human, a product, an emulsion or a vaccine of the invention. The disease linked to an over-production of TNFα may be selected from the group consisting of ankylosing spondylitis, psoriasis, rhumatoïd arthritis, Juvenile idiopathic arthritis, Inflammatory Bowel Disease, Crohn's disease, cachexia, and cancer.

## DETAILED DESCRIPTION

In a first aspect, this invention thus includes an immunogenic product comprising TNFα coupled with KLH, wherein the TNFα is strongly inactivated, which means that the product shows less than 30%, preferably 25%, more preferably 20%, more preferably 15%, even more preferably 10% of cytolytic activity in the conditions of hereunder cited TEST A; an emulsion comprising said product with combination to an oil and a surfactant; and a vaccine composition comprising said emulsion or said product.

As used herein, "TNFα" encompasses any TNFα originating from a mammalian organism. Mammalian TNFα encompasses human TNFα, equine TNFα, cat TNFα, dog TNFα, bovine TNFα, ovine TNFα, as well as caprine TNFα, which are all well known from the one skilled in the art, the corresponding amino acid sequences and nucleic acid sequences encoding them being publicly available for a long time, including in various nucleic acid and amino acid sequences databases. Illustratively, the amino acid sequences of various mammal TNFα are referred to in the GenBank database and in the NCBI (National Center for Biology Information) database , including : human TNFα (Genbank # CAA26669), murine TNFα (Genbank CAA68530), dog TNFα (Genbank # ABJ51909), equine TNFα (NCBI # NP-001075288), cat TNFα (NCBI # NP-001009835), bull TNFα (NCBI # NP-776391), porcine TNFα (NCBI # NP-001166496), goat TNFα (NCBI # AAF87741), rat TNFα (NCBI # NP036807), sheep TNFα (NCBI # NP-001020031).

According to an embodiment, the TNFα is a human TNFα molecule. Human TNFα consists of a homotrimeric TNFα molecule that is formed by the association of three TNFα molecules of approximately 17 kDa (17.35 kDa).

According to the invention, test A is used to determine the percentage of inactivation of human TNFα bioactivity in the product of the invention. The test is based on the cytolysis of murine L929 cells induced by human TNFα in the presence of Actinomycin D. This test is carried out at T0, i.e. the product is in liquid form and stored at 4°C for less than 10 days after production.

Test A is carried out according to the following method:

L929 mouse fibroblasts cells (Sigma n°85011425) are plated at 1.5 $10^4$/cm$^2$ in Culture Medium (DMEM (Cambrex BE12604F) supplemented 10% FBS (Sigma F7524), 2 mM glutamine (Sigma G7513), 100 U/ml penicillin/streptomycin (Sigma P0781) and 1 mM Sodium Pyruvate (Sigma S8636)) and cultured for 2 days at 37°C 5% $CO_2$ to obtain a subconfluent monolayer.

L929 cells are then harvested and plated in 96 well flat bottom culture plates at 2 $10^4$ cells/well in 100 μl of Plating Medium (DMEM F12 (Cambrex BE12719F) supplemented with 2% FBS, 2 mM glutamine, 100 U/ml penicillin/streptomycin and 1 mM Sodium Pyruvate) and cultured for 21 +/- 1 h at 37°C, 5%$CO_2$.

A series of ten two-fold dilutions of the product of the invention is prepared from 120 μl of the product of the invention at 6400 ng/ml TNFα equivalent diluted in 60 μl of Assay Medium (HL1 (Cambrex US77201) supplemented with 2 mM glutamine, 100 U/ml penicillin/streptomycin and 1 mM Sodium Pyruvate).

The concentration unit used may be TNFα equivalent concentration (Example 3) or total proteins determined using a BCA test (Example 12). TNFα equivalent concentration unit makes it possible to compare different batches, with the same TNF content, in cellular bioassay and *in vivo* in the TNFα shock model. A concentration in TNFα equivalent is determined as following:

[TNFα equivalent concentration] = (quantity of TNFα at the beginning of the process)-10%.

If a final step of filtration with a cut-off of 300 kDa has been carried out in the process for preparing the product of the invention, 75 % of TNFα is removed (as evidenced on a radioactive test in which TNFα was radio-labeled) and the concentration in TNFα equivalent is determined as following: [TNFα equivalent concentration] = [(quantity of TNFα at the beginning - 10%) - 75 %]. Of note, yield is consistent during manufacturing process. A series of ten three-fold dilutions of the standard (human TNFα 6.24 mg/ml, Boehringer ingelheim 03030R1) is prepared from 120 μl of human TNFα at 8 ng/ml in 60 μl of Assay Medium. EC50 of TNF from Boehringer ranges from 10 to 500 pg/ml.

At the end of culture time of L929 cells, cells should be subconfluent. The wells of the flat-bottom culture plates are then emptied of the culture medium and 50 μl of each dilution are transferred into the wells of the flat-bottom culture plate.

50 μl of Assay Medium supplemented with Actinomycin D at 2 μg/ml (Sigma A9415) are added to each well.

The L929 cells are then cultured for 20 +/- 1 h at 37°C 5% $CO_2$.

At the end of the culture, viability of the L929 cells is assessed using methods well-known in the art. One example of said methods is the following: 20 μl/well of a solution of MTS/PMS (100 μl MTS/5 μl PMS; Promega G5430) are added to the wells and the plate is incubated for another 4h at 37°C 5% $CO_2$. The plate is then read at 490 nm on a spectrophotometer.

The percentage of viability is calculated as following:

$$\% = 1-[(OD_{product}-OD_{TNFstandard})/(OD_{cells}- OD_{TNFstandard})]$$

$OD_{product}$ stands for the optical density of well with the product of the invention.

$OD_{TNFstandard}$ stands for the optical density of well with the standard TNFα at 200 ng/ml.

$OD_{cells}$ stands for the optical density of control well with no standard nor product of the invention.

The person skilled in the art can thus determine from Test A the percentage of cytolytic activity for the tested product at 100 ng/ml, 200 ng/ml, 400 ng/ml and 800 ng/ml TNFα equivalent.

Test A is carried out in Example 3 and 12 as shown hereafter.

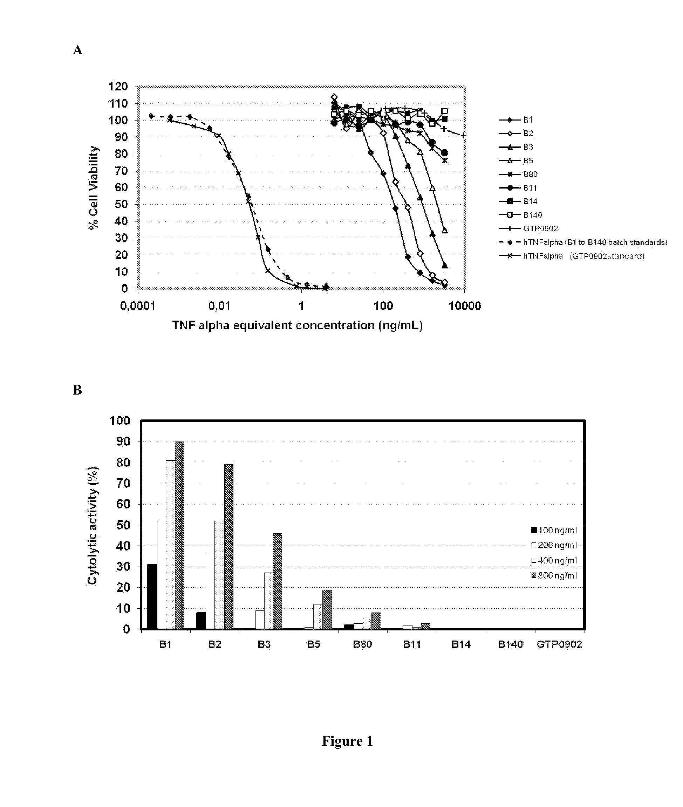

In one embodiment of the invention, the product at a concentration of 100 ng/ml TNFα equivalent, preferably 200 ng/ml TNFα equivalent, more preferably 400 ng/ml TNFα equivalent, even more preferably 800 ng/ml TNFα equivalent, kills less than 30 % of L929 cells (which means that more than 70% of L929 cells are viable), preferably less than 25% (which means that more than 75% of L929 cells are viable), more preferably less than 20% (which means that more than 80% of L929 cells are viable), more preferably less than 15% of L929 cells (which means that more than 85% of L929 cells are viable), even more preferably less than 10% of L929 cells (which means that more than 90% of L929 cells are viable) (see Figure 1B).

Test A as described here above can also be used to determine the EC50 of the product and the Inactivation Factor of the product. The EC50 corresponds to the concentration of the product necessary to kill 50% of L929 cells. The Inactivation Factor can be calculated as following: $EC50_{product}/EC50_{TNF\alpha}$.

In an embodiment of the invention, the product presents an EC50 which is more than 500, preferably more than 1000, preferably more than 2000, more preferably more than 3000, even more preferably more than 5000 ng/ml.

In another embodiment of the invention, the product presents an Inactivation Factor that is more than 15000, preferably more than 30000, even more preferably more than 50000. In one embodiment of the invention, the Inactivation Factor of the product is more than 100000.

This invention goes even further in inactivation, and ensures that the vaccine of the invention, in the conditions of temperature of the human body, i.e. *in vivo* temperature conditions, typically at 37°C., will remain inactive during the necessary time, i.e. the time during which the immunization has to be effective. In this regard, Test B was designed, in conformity with the European and American Pharmacopeia. In the meaning of this invention, the terms "remain inactive" or "inactive overtime", mean that the product shows less than 80% of cytolytic activity in the conditions of TEST B.

According to the invention, test B is used to determine the percentage of inactivation of human TNFα bioactivity in the product of the invention, when placed in the conditions of temperature of the human body. The test is based on the cytolysis of murine L929 cells induced by human TNFα in the presence of Actinomycin D, and is carried out at T6, i.e. the product is in liquid form and stored at 37°C for 6 weeks.

Test B is carried out according to the following method: L929 mouse fibroblasts cells (Sigma n°85011425) were plated at $1.5 \ 10^4/cm^2$ in Culture Medium (DMEM (Cambrex BE12604F) supplemented 10% FBS (Sigma F7524), 2 mM glutamine (Sigma G7513), 100 U/ml penicillin/streptomycin (Sigma P0781) and 1 mM Sodium Pyruvate (Sigma S8636)) and cultured for 2 days at 37°C 5% $CO_2$ to obtain a subconfluent monolayer.

L929 cells were then harvested and plated in 96 well flat bottom culture plates at $2 \ 10^4$ cells/well in 100 μl of Plating Medium (DMEM F12 (Cambrex BE12719F) supplemented with 2% FBS, 2 mM glutamine, 100 U/ml penicillin/ streptomycin and 1 mM Sodium Pyruvate) and cultured for 21 +/- 1 h at 37°C, 5% $CO_2$.

A series of five three-fold dilutions of the product of the invention was prepared from 120 μl of the product of the invention at 6400 ng/ml diluted in 60 μl of Assay Medium (HL1 (Cambrex US77201) supplemented with 2 mM glutamine, 100 U/ml penicillin/streptomycin and 1 mM Sodium Pyruvate).

The concentration unit used may be TNFα equivalent concentration (Example 4) or total proteins determined using a BCA test (Example 12). TNFα equivalent concentration makes it possible to compare different batches, with the same TNFα content, in cellular bioassay and *in vivo* in the TNF shock model. A concentration in TNFα equivalent is determined as following:

[TNFα equivalent concentration] = (quantity of TNFα at the beginning of the process)-10%. If a final step of filtration with a cut-off of 300 kDa has been carried out in the process for preparing the product of the invention, 75 % of TNFα is removed (as evidenced on a radioactive test in which TNFα was radio-labeled) and the concentration in TNFα equivalent is determined as following: [TNFα equivalent concentration] = [(quantity of TNFα at the beginning - 10%) - 75 %]. Of note, yield is consistent during manufacturing process. A series of ten three-fold dilutions of the standard (human TNFα 6.24 mg/ml, Boehringer ingelheim 03030R1) was prepared from 120 μl of human TNFα at 8 ng/ml in 60 μl of Assay Medium. EC50 of TNF from Boehringer ranges from 10 to 500 pg/ml.

At the end of culture time of L929 cells, cells were subconfluent. The wells of the flat-bottom culture plates were then emptied of the culture medium and 50 %l of each dilution were transferred into the wells of the flat-bottom culture plate.

50 μl of Assay Medium supplemented with Actinomycin D at 2 μg/ml (Sigma A9415) were added to each well.

The L929 cells were then cultured for 20 +/- 1 h at 37°C 5% $CO_2$.

At the end of the culture, viability of the L929 cells is assessed using methods well-known in the art. One example of said methods is the following: 20 μl/well of a solution of MTS/PMS (100 μl MTS/5 μl PMS; Promega G5430) are added to the wells and the plate is incubated for another 4h at 37°C 5% $CO_2$. The plate is then read at 490 nm on a spectrophotometer.

The percentage of viability is calculated as following:

$$\% = 1 - [(OD_{product} - OD_{TNFstandard})/(OD_{cells} - OD_{TNFstandard})]$$

$OD_{product}$ stands for the optical density of well with the product of the invention.

$OD_{TNFstandard}$ stands for the optical density of well with the standard TNFα at 200 ng/ml.

$OD_{cells}$ stands for the optical density of control well with no standard nor product of the invention.

The person skilled in the art can thus determine from Test B the percentage of cytolytic activity of the tested product remaining after 6 weeks at 37°C. Test B is carried out in Example 4 and Example 12 as shown hereafter.

In one embodiment of the invention, the product at a concentration of 100 ng/ml TNFα equivalent kills less than 80 % of L929 cells (which means that more than 20% of L929 cells are viable), preferably less than 70% (which means that more than 30% of L929 cells are viable), more preferably less than 60% (which means that more than 40% of L929 cells are viable), even more preferably less than 50% (which means that more than 50% of L929 cells are viable).

In one embodiment of the invention, the product at a concentration of 350 ng/ml TNFα equivalent kills less than 90 % of L929 cells (which means that more than 10% of L929 cells are viable), preferably less than 80% (which means that more than 20% of L929 cells are viable), more preferably less than 70% (which means that more than 30% of L929 cells are viable), more preferably less than 60% (which means that more than 40% of L929 cells are viable) and even more preferably less than 50% (which means that more than 50% of L929 cells are viable).

In one embodiment of the invention, the product at a concentration of 1000 ng/ml TNFα equivalent kills less than 90 % of L929 cells (which means that more than 10% of L929 cells are viable), preferably less than 80% (which means that more than 20% of L929 cells are viable), more preferably less than 70% (which means that more than 30% of L929 cells are viable).

Test B as described here above can also be used to determine the EC50 of the product and the Inactivation Factor of the product. The EC50 corresponds to the concentration of the product necessary to kill 50% of L929 cells after 6 weeks of storage at 37°C. The Inactivation Factor can be calculated as following: $EC50_{product}/EC50_{TNFα}$.

In an embodiment of the invention, the product when placed 6 weeks at 37°C presents an EC50 which is more than 100, preferably more than 250, more preferably more than 500 ng/ml.

In another embodiment of the invention, the product when placed 6 weeks at 37°C presents an Inactivation Factor that is more than 500, preferably more than 2000, more preferably more than 5000, even more preferably more than 10000.

According to an embodiment, the product of the invention may comprise free TNFα homopolymers. In a preferred embodiment, said TNFα homopolymers have a molecular weight of more than 100 kDa, preferably of more than 300kDa. In an embodiment, the percentage of free TNFα homopolymers of more than 100kDa, preferably of more than 300kDa, is of less than 30% w/w of total TNFα.

The percentage of free TNFα homopolymers may be determined according to Test C.

Test C is based (1) on purification of free TNFα or KLH homopolymers by an immunocapture step using magnetic beads coated with anti-TNFα monoclonal antibodies or anti-KLH polyclonal antibodies respectively and (2) quantification of free TNFα or KLH homopolymers by specific ELISA.

According to test C, beads are coated with anti-KLH or anti-TNFα antibodies are prepared (an example of such preparation is explained in Example 5). Coated and non-coated beads are mixed with the product and incubated during 12-16h at 4°C. The surpernatant is then harvested using the magnet and analyzed by ELISA.

Three ELISA are then performed:

- a KLH-KLH ELISA where the capture antibody and the primary antibody are an anti-KLH antibody,
- a TNF-TNF ELISA where the capture antibody and the primary antibody are an anti-TNFα antibody,
- a KLH-TNF ELISA where the capture antibody is an anti-KLH antibody and the primary antibody is an anti-TNFα antibody or inversely.

The ELISA are developed by any colorimetric means known in the art such as for example using detection antibody labelled with biotin, a poly-streptavidin HRP amplification system and an o-phenylenediamine dihydrochloride substrate solution.

Analysis of the results of the ELISA allows the determination of the percentage of free TNFα homopolymers by comparison with total TNFα present in the product of this invention as shown in Example 5.

In a more preferred embodiment, the product is free of TNFα homopolymers having a molecular weight of less than 100 kDa (which is the apparent molecular mass of dimers of the homotrimeric TNFα molecule). In a more preferred embodiment, the product is free of TNFα oligomers having a molecular weight of less than 300 kDa (which is the apparent molecular mass of hexamers of the homotrimeric TNFα molecule). Without willing to be linked by any theory, the Applicant suggests that removing the TNFα oligomers of less than 100 kDa, and in an embodiment, of less than 300 kDa, may increase the safety of the product for human and non-human mammal uses and improve the immunogenic properties of the final immunogenic product.

*[Emulsion and vaccine composition containing such emulsion]*

This invention also relates to a formulation of the product of the invention, wherein the product is within an emulsion. Advantageously, the vaccine composition of the invention comprises or consists of said emulsion. Such emulsion comprises the immunogenic product of the invention, an oil and a surfactant or a mixture of at least one oil and at least one surfactant. Preferably, the oil or the mixture oil/surfactant is a pharmaceutically acceptable excipient. More preferably, the mixture of oil and surfactant is an adjuvant, even more preferably an immunoadjuvant. Preferred adjuvant is ISA 51. The emulsion of the invention may be a water-in-oil emulsion or an oil-in-water emulsion.

In another embodiment, the amount of the immunogenic product according to the invention of more than 0.01% (w/w) and less than 1% (w/w) of the total weight of the said emulsion.

*[Adjuvants]*

The emulsion or the vaccine composition of the invention may comprise adjuvant, especially immunoadjuvants. In an embodiment, the amount of adjuvant ranges from 0.00001% (w/w) to 1%, preferably 0.0001 to 0.1%, more preferably from 0,001 to 0.01 % (w/w) of the total weight of the vaccine composition.

Any suitable adjuvant known by the skilled artisan may be used in the vaccine composition above, including oil-based adjuvants such as for example Freund's Incomplete Adjuvant, mycolate-based adjuvants (e.g., trehalose dimycolate), bacterial lipopolysaccharide (LPS), peptidoglycans (i.e., mureins, mucopeptides, or glycoproteins such as N-Opaca, muramyl dipeptide [MDP], or MDP analogs), MPL (monophosphoryl lipid A), proteoglycans (e.g., extracted from Klebsiella pneumoniae), streptococcal preparations (e.g., OK432), Biostim.TM.(e.g., 01 K2), the "Iscoms" of EP 109 942, EP 180 564 and EP 231 039, aluminum hydroxide, saponin, DEAE-dextran, neutral oils (such as miglyol), vegetable oils (such as arachid oil), liposomes, Pluronic.RTM. polyols, the Ribi adjuvant system (see, for example GB-A-2 189 141), or interleukins, particularly those that stimulate cell mediated immunity. An alternative adjuvant consisting of extracts of Amycolata, a bacterial genus in the order Actinomycetales, has been described in U.S. Pat. No. 4,877,612. Additionally, proprietary adjuvant mixtures are commercially available. The adjuvant used will depend, in part, on the recipient organism. The amount of adjuvant to administer will depend on the type and size of animal. Optimal dosages may be readily determined by routine methods.

Oil adjuvants suitable for use in water-in-oil emulsions may include mineral oils and/or metabolizable oils. Mineral oils may be selected from Bayol®., Marcol.®. and Drakeol, including Drakeol® 6VR (SEPPIC, France). ®. Metabolisable oils may be selected from SP oil (hereinafter described), Emulsigen (MPV Laboratories, Ralston, NZ), Montanide 264,266,26 (Seppic SA, Paris, France), as well as vegetable oils, such as peanut oil and soybean oil, animal oils such as the fish oils squalane and squalene, and tocopherol and its derivatives.

In addition, the adjuvant may include one or more wetting or dispersing agents in amounts of about 0.1 to 25%, more preferably about 1 to 10%, and even more preferably about 1 to 3% by volume of the adjuvant. Particularly preferred as wetting or dispersing agents are non-ionic surfactants. Useful non-ionic surfactants include polyoxyethylene/polyoxypropylene block copolymers, especially those marketed under the trademark Pluronic®. and available from BASF Corporation (Mt. Olive, N.J.). Other useful nonionic surfactants include polyoxyethylene esters such as polyoxyethylene sorbitan monooleate, available under the trademark Tween 80®. It may be desirable to include more than one, e.g. at least two, wetting or dispersing agents in the adjuvant as part of the vaccine composition of the invention.

Suitable adjuvants may include but are not limited to surfactants known by one skilled in the art, such as for example hexadecylamine, octadecylamine, lysolecithin, dimethyldioctadecylammonium bromide, N,N-dioctadecyl-N'-N-bis(2- hydroxyethyl-propane di-amine), methoxyhexadecyl-glycerol, and pluronic polyols; polanions, e.g., pyran, dextran sulfate, poly IC, polyacrylic acid, carbopol; peptides, e.g., muramyl dipeptide, aimethylglycine, tuftsin, oil emulsions, alum, and mixtures thereof. Other potential adjuvants include the B peptide subunits of E. coli heat labile toxin or of the cholera toxin. McGhee, J. R., et al., "On vaccine development," Sem. Hematol., 30:3-15 5 (1993).

*[Further surfactants]*

In the embodiments of a vaccine composition according to the invention comprising an emulsion, the vaccine composition preferably contains, in addition to the combination of the immunogenic product and the one or more oily immunoadjuvant substances, also one or more surfactant agents. Illustrative embodiments of surfactive agents include mannide monoleate such as Montanide® 80 marketed by Arlacel (SEPPIC, France).

In an embodiment, the amount of surfactant agent ranges from 0.00001 % (w/w) to 1%, preferably 0.0001 to 0.1 %, more preferably from 0,001 to 0.01% (w/w) of the total weight of the vaccine composition.

*[Lyophilized products]*

According to an embodiment and for storage purposes, the product or the vaccine composition of the invention may be lyophilized. Vaccine compositions may thus be presented in a freeze-dried (lyophilized) form. In said embodiment, the immunogenic product according to the invention is combined with one or more lyophilisation auxiliary substances. Various lyophilisation auxiliary substances are well known by the one skilled in the art. Lyophilization of auxiliary substances encompasses sugars like lactose and mannitol.

In such embodiment where the vaccine composition consists of a lyophilised composition for use as a liquid emulsion comprising a surfactant agent, the vaccine composition preferably comprises an amount of the immunogenic product according to the invention of more than 0.1% (w/w) and less than 10% (w:/w) of the total weight of the said vaccine composition.

*[Stabilizers]*

In some embodiments, the vaccine may be mixed with stabilizers, e.g. to protect degradation-prone proteins from being degraded, to enhance the shelf-life of the vaccine, or to improve freeze-drying efficiency. Useful stabilisers are i.a, SPGA (Bovarnik et al; J. Bacteriology 59: 509 (1950)), carbohydrates e.g. sorbitol, mannitol, trehalose, starch, sucrose, dextran or glucose, proteins such as albumin or casein or degradation products thereof, and buffers, such as alkali metal phosphates.

*[Administration route]*

The vaccine compositions according to the invention may be administered to the subject to be immunized by any conventional method including, by injectable, e.g. intradermal, intramuscular, , intraperitoneal, or subcutaneous injection; or by topical, such as for example by .transdermal delivery. The treatment may consist of a single dose or a plurality of doses over a period of time.

*[Dosage form]*

The forms suitable for injectable use may include sterile solutions or dispersions and sterile powders for the extemporaneous preparation of sterile injectable solutions or dispersions. The prevention against contamination by microorganisms can be brought about by adding in the vaccine composition various antibacterial and antifungal agents, for example, parabens, chlorobutanol, phenol, sorbic acid, thimerosal and the like. In many cases, it may be preferable to include isotonic agents, for example, sugars or sodium chloride. Prolonged absorption of the injectable compositions can be brought about by the use in the compositions of agents delaying absorption, for example, aluminium monostearate and gelatine.

According to an embodiment, a lyophilized vaccine composition, of the invention is solubilized in water for injection and gently mixed; then an immunoadjuvant, preferably ISA 51, is added; the mixture is gently mixed for emulsification and charged into a suitable syringe. This invention thus also relates to a medical device, including a syringe filled or prefilled with a vaccine composition of the invention. The emulsion is ideally prepared extemporaneously. However, the syringe containing the emulsion can be stored less than 10 hours at 2 - 8 °C. In this case, the emulsion should be allowed to warm up before injecting by friction between the hands.

*[Unit dosage range]*

Preferably, when human use or non-human mammal use is sought, a dosage unit of a vaccine composition according to the invention preferably comprises an amount of the immunogenic product ranging from 0.1 to 1000 $\mu$g when designed for animals, and ranging from 20 to 1000 $\mu$g when designed for humans.

Preferably, when human use is sought, a typical dosage unit of a vaccine composition according to the invention preferably comprises an amount of the immunogenic product ranging from 20 $\mu$g to 1000 $\mu$g, most preferably ranging from 25 $\mu$g to 600 $\mu$g.

*[Mechanism of action]*

The present invention also relates to a method for preparing an immunogenic product that is useful for inducing an immune response in a mammal to whom said immunogenic product is administered, including a humoral immune response wherein antibodies that neutralize the immmunosuppressive, apoptotic or angiogenic properties of the endogenous cytokine.

The produced immunogenic product is mainly used in vaccine compositions for preventing or treating a disease linked to an over-production of TNFα. More specifically, this invention relates to a method for preventing or treating a disease linked to an over-production of TNF comprising a step of administering to the animal, including a human, a therapeutically affective amount of a product, emulsion or vaccine of the invention. The disease linked to an over-production of TNFα may be selected from the group consisting of ankylosing spondylitis, psoriasis, rhumatoïd arthritis, Juvenile idiopathic arthritis, Inflammatory Bowel Disease, Crohn's disease, cachexia, and cancer. One object of the invention is the product, emulsion or vaccine of the invention as described here above for use in preventing or treating a disease linked to an over-production of TNFα.

A further aspect of the present invention therefore relates to the use of an immunogenic product or of a vaccine composition as defined above. A further object of the invention consists of a method for inducing the production of antibodies that neutralize the activity of endogeneous TNFα in a mammal, comprising a step of administering to said mammal (i) a vaccine composition as disclosed above or (ii) an immunogenic product as described above together with one or more immunoadjuvants.

### [Kit and Medical device]

This invention also pertains to a kit comprising:

- 1 vial (Vial Number 1) containing lyophilized product of the invention, typically of 3mL;
- 1 vial (Vial Number 2) containing water for injection typically of 2mL;
- 1 vial (Vial Number 3) containing adjuvant, preferably ISA51; this vial is capable of containing 3 mL of adjuvant and may be a container of 8 mL;
- 1 syringe, typically a Braun Injekt-F® of 1 mL;
- 1 needle (Needle Number 1) for emulsion preparation; this needle is preferably a 20G needle;
- 1 needle (Needle Number 2) for injection, preferably intramuscular injection; this needle is preferably a 23G needle.

This invention also pertains to a method for preparing a vaccine from the kit, comprising:

(1) injecting of water for injection from Vial Number 2 into the Vial Number 1 by using the syringe connected to Needle number 1;
(2) rotating gently Vial Number 1 during 1-5 minutes until complete solubilization of the preparation ;
(3) with the same syringe and needle, pulling up adjuvant from Vial Number 3. Discharge this syringe content into Vial Number 1.
(4) pumping up and down the total vial content a sufficient number of times for emulsifying the content, typically 30 times and finally pulling up the whole emulsion.

Prior to injection, Needle Number 1 is preferably switched for Needle Number 2 and air is purged from the syringe.

This invention also relates to the medical device which is the syringe filled or prefilled with the vaccine composition of the invention.

The invention also relates to a medical device comprising a vial or a carpule prefilled with the product of the invention or with the vaccine composition of the invention.

This invention also relates to two methods (hereinafter "main method" and "variant method") for preparing a product comprising TNFα coupled with KLH, wherein the TNFα is strongly inactivated, which means that the product shows less than 30%, preferably 25%, more preferably 20%, more preferably 15%, even more preferably 10 % of cytolytic activity or presents an inactivation factor of more than 15000, in the conditions of TEST A.

In both methods, preferably, the TNFα starting product consists of a recombinant human TNFα that may be obtained by various methods described in the art. Illustratively, TNFα consists of a recombinant human TNFα that is produced by *E. coli* cells that have been transformed by a plasmid having inserted therein an expression cassette encoding human TNFα. Most preferably, the TNFα starting product does not contain a detectable amount of endotoxin. For use in the method of the invention, TNFα is preferably in a liquid solution, preferably a buffer solution having a pH ranging from 6.5 to 7.5. In some embodiments, the liquid solution containing TNFα also contains DMSO (dimethylsulfoxide), preferably at a final concentration ranging from 0.1% (w/w) to 5% (w/w), and most preferably from 0.5% (w/w) to 3% (w/w). DMSO is a well known anti-oxidant compound susceptible of increasing the availability of the glutaraldehyde-reactive groups present in the TNFα molecule. In some embodiments, the liquid solution containing TNFα also contains EDTA at a final concentration ranging from 1 mM to 20 mM, preferably from 3 mM to 10 mM.

Preferably, the KLH starting product consists of a highly purified KLH extracted from the lymph of the marine gastropod mollusk *Megathura cremulata,* and the said KLH starting product preferably does not contain a detectable amount of endotoxin. Naturally produced KLH generally consists of a di-decamer structure (non covalent tubular assembly of 20 subunits), each decamer unit consisting of a homopolymer of subunits KLH1 or KLH2. Preferably, the KLH di-decamer has a molecular weight (MW) of approximately $8.10^6$ Da, it being taken into account that the molecular weight of a KLH1 subunit is of about 350 kDa and that the molecular weight of a KLH2 subunit is of about 390 kDa.

*Reaction TNFa+ KLH + glutaraldehyde*

In a first embodiment, the method of the invention comprises:

- a first step (step a) of mixing together (i) purified TNF$\alpha$, (ii) purified Keyhole limpet hemocyanin and (iii) glutaraldehyde.

Due to the reaction of glutaraldehyde with the free amino groups borne by both KLH and TNF$\alpha$, the product which is obtained at the end of step a) comprises monomers and oligomers of KLH having TNF$\alpha$ molecules associated therewith, where TNF$\alpha$ molecules include (i) TNF$\alpha$ monomers and (ii) TNF$\alpha$ oligomers.

In a preferred embodiment of step a), TNF$\alpha$ and KLH are firstly mixed together in the appropriate amounts, before adding glutaraldehyde.

In some embodiments, TNF$\alpha$ and KLH are mixed at step a) at a TNF$\alpha$:KLH molar ratio ranging from 10:1 to 40:1. In some preferred embodiments, TNF$\alpha$ and KLH are mixed at step a) at a TNF$\alpha$:KLH molar ratio ranging from 30:1 to 40:1.

In some preferred embodiments, TNF$\alpha$ and KLH are mixed at step a) at a TNF$\alpha$:KLH molar ratio ranging from 35:1 1 to 40:1.

In some embodiments of step a), hereinafter referred as step al), glutaraldehyde is used at a final concentration in the reaction mixture ranging from 1 mM to 50 mM, preferably from 20 mM to 30 mM, more preferably at 25 mM. In some embodiments of step a), glutaraldehyde is incubated with TNF$\alpha$ and KLH for a period of time ranging from more than 110 min to less than 400 min, preferably about 130, 140, 150, 160, 170, 180, 190, 200, 210, 220, 230 and 240 minutes. In an embodiment, glutaraldehyde is added at 25 mM during about 120 minutes. In another embodiment, glutaraldehyde is added at 25 mM during about 240 minutes.

Advantageously, step a) of incubation with glutaraldehyde is performed at a temperature ranging from 18°C to 37°C, preferably from 18°C to 27°C.

*Quenching after glutaraldehyde reaction*

According to an embodiment, the reaction with glutaraldehyde may be stopped by adding a quenching compound, preferably a quenching compound that is selected from (i) a reducing agent and (ii) an amino acid selected from the group consisting of lysine and glycine and mixture thereof.

The reducing agent may consist of any one of the reducing agents known in the art which, due to their reducing properties, have the ability to reduce the remaining free aldehyde groups of glutaraldehyde that have not reacted with either TNF$\alpha$ or KLH free amino groups. The reducing agent may be selected from the group consisting of sodium borohydride, sodium cyanoborohydride.

According to an embodiment, in the embodiments wherein the said quenching compound is an amino acid, the said amino acid consists of glycine. In some embodiments of step b) where glycine and/or lysine are used for blocking the reaction with glutaraldehyde, the selected amino acid is used at a final concentration in the reaction mixture ranging from 0.01 M to 1 M, preferably from 0.05 M to 0.5 M, and most preferably from 0.08 M to 0.2 M, e.g. at 0.1 M as shown in the examples herein. In an embodiment, incubation with the quenching compound is performed for a period of time ranging from 1 minute to 120 minutes, preferably from 5 minutes to 60 minutes, e.g. for 15 minutes as shown in the examples herein. In another embodiment, this step is performed at a temperature ranging from 20°C to 30°C, preferably from 23°C to 27°C.

*Step b) of the method*

In this first embodiment, the method of the invention comprises, after step a) is carried out, optionally followed by the above-mentioned quenching reaction, a step b, which is as follows:

b) removing compounds having a molecular weight of less than 10 kDa

At step b), the small compounds of less than 10 kDa that are present in the reaction mixture are removed. These small compounds encompass mainly the excess glutaraldehyde and the excess quenching compound molecules that have not reacted with TNF$\alpha$ nor KLH, as well as eventual protein degradation products of a size smaller than endogeneous TNF$\alpha$ or native KLH.

Step b) may be performed according to any known technique which allows removing compounds of less than 10 kDa, which techniques include dialysis with a dialysis membrane having a cut-off of 10kDa or filtration using a filtration membrane having a cut-off of 10 kDa. Illustratively, step b) may consist of a step of tangential flow filtration using a filtration membrane having a cut-off of 10 kDa, as it is shown in the examples herein. The filtration retentate, which is devoid of the undesirable small compounds, is collected at the end of step b).

If desired, step b) may comprise a preliminary step of removing the eventual compound aggregates present in the reaction mixture obtained at the end of step b). The said preliminary step may consist of a conventional filtration step for removing solid aggregates eventually present in suspension in a liquid solution, e.g. a filtration step using an appropriate filtration membrane, e.g. a filtration membrane having a pore size of 0.2 $\mu$m.

### *Step c) of the method*

In this first embodiment, the method of the invention comprises, after step b) is carried out, the following step c):

c) adding formaldehyde in a concentration/time of reaction condition ranging from at least 60 mM for at least 240 hours to at least 120 mM for at least 144 hours.

Step c) consists of adding formaldehyde at specified concentrations and specified periods of time. The intermediate product obtained at the end of step c) is subjected to a formaldehyde treatment at a concentration within the reaction mixture of at least 60 mM, preferably of 60 to 500 mM, more preferably of 100 to 300 mM for at least 10 days, preferably for 240 to 500 hours, more preferably for 288 to 336 hours. In an embodiment, the intermediate product obtained at the end of step c) is subjected to a formaldehyde treatment at a concentration within the reaction mixture of at least 120 mM, preferably 120 to 270 mM for at least 6 days (144 hours), preferably for 144 to 500 hours, more preferably for 144 to 360 hours.

In an embodiment, in step c) the formaldehyde treatment is performed at a concentration in the mixture of 220 mM to 270 mM during a period of time of more than 300 hours. In an embodiment, the period of time is of more than 310, 320 and 330 hours, e.g. a period of time of 336 hours (14 days) as it is shown in the examples herein. Preferably the period of time of treatment with formaldehyde preferably does not exceed a period of time of 500 hours, which encompasses periods of time of less than 490, 480, 470, 460, 450, 440, 430, 420, 410, 400, 390, 380, 370 and 360 hours.

At step c), the concentration of formaldehyde within the reaction mixture is preferably of more than 200 mM. A concentration of formaldehyde of more than 200 mM especially encompasses a concentration of more than 220, 230, 240 and 250 mM. In an embodiment, the concentration of formaldehyde is less than 270 mM. In a preferred embodiment, formaldehyde is applied at a final concentration of at least 200 mM during at least 240 hours, preferably of 220 mM to 270 mM, preferably 250 mM, for at least 300 hours.

At step c), incubation with formaldehyde is performed preferably at a temperature ranging from 30°C to 42°C, e.g. at 37°C as it is shown in the examples herein.

As it was expected from the prior art knowledge, said formaldehyde treatment causes significant structural changes to the product. Thus, it was all the more surprising that, despite this treatment, the immunogenic product that is obtained by the method according to the invention is endowed with expected anti-TNF$\alpha$ immunogenic properties.

### *Step d) of the method*

At step d) of the method, the reaction with formaldehyde is stopped by adding a quenching compound, preferably a quenching compound that is selected from (i) a reducing agent and (ii) an amino acid selected from the group consisting of lysine and glycine.

The reducing agent may consist in any one of the reducing agents known in the art which, due to their reducing properties, reduce the remaining free aldehyde groups of formaldehyde that have not reacted with either TNF$\alpha$ or KLH free amino groups.

The reducing agent may be selected from the group consisting of sodium borohydride, sodium cyanoborohydride.

According to an embodiment, in the embodiments wherein the said quenching compound is an amino acid, the said amino acid consists of glycine. In some embodiments of step b) where glycine and/or lysine are used for blocking the reaction with formaldehyde, the selected amino acid is used at a final concentration in the reaction mixture ranging from 0.01 M to 1.5 M, preferably from 0.05 M to 1 M, and most preferably from 0.2 M to 0.8 M, e.g. at 0.38 M as shown in the examples herein. In an embodiment, incubation with the quenching compound is performed for a period of time ranging from 5 minutes to 120 minutes, preferably from 10 minutes to 80 minutes, e.g. for 60 minutes as shown in the examples herein. In another embodiment, this step is performed at a temperature ranging from 18°C to 30°C, preferably from 19°C to 27°C.

### Removal of species of less than 100kDa, preferably of less than 300 kDa

After step d), the collection of the product of the invention may be performed.

However, according to a very preferred embodiment, after step d) and prior to collecting the product, a further step is performed. This step consists of removing substances having a molecular weight of less than 100, pref 300 kDa. Removal of substances having a molecular weight of less than 300 kDa may be performed by the skilled artisan by any technique known in the art for removing substances having a molecular weight of more than 300 kDa from a liquid solution. In a first embodiment, the technique used is a filtration step that is performed by using a filtration membrane having a cut-off value of at least 100 kDa, or in an embodiment of at least 300 kDa, which encompasses an ultrafiltration step or a tangential filtration step. In a second embodiment, the technique used consists of a tangential filtration step using a filtration membrane having a cut-off value of at least 100 kDa, which includes a cut-off value of at least 300 kDa.

Without willing to be linked by any theory, the Applicant noticed that, surprisingly, performing this step was beneficial to the product. Especially, this step removed homopolymers of TNFα, which have not reacted with KLH. It was observed that more than 50% of initial TNFα may be removed in this step of the process and that, unexpectedly, the remaining product was even better as far as immunogenicity was concerned.

### Lyophilisation

Optionally, the final immunogenic product according to the invention may be further processed for long term storage before use. The inventors have shown that lyophilisation of the product of the invention may improve its stability upon long term storage and may improve the irreversibility of the TNFα biological inactivation. The lyophilised immunogenic product according to the invention may be stored unaltered for months, including for at least 6 months, in sterile and apyrogenic closed recipients at a temperature from about 2°C to about 25°C until its use.

### Alternative method

In a variant of the invention, the method for preparing a product comprising TNFα coupled with KLH, wherein the TNFα is strongly inactivated, which means that the product shows less than 30%, preferably 25%, more preferably 20%, even more preferably 15% of cytolytic activity in the conditions of TEST A, comprises the steps of:

a) mixing together (i) purified TNFα, (ii) purified Keyhole limpet hemocyanin and (iii) glutaraldehyde
b) removing compounds having a molecular weight of less than 10 kDa and is characterized by a specific embodiment of step a), hereinafter referred to as step a2) where glutaraldehyde is applied during more than 18 hours, or more than 20, or more than 24 hours, at a concentration of at least 20mM , the reaction with glutaraldehyde is stopped by adding a quenching compound, preferably a quenching compound that is selected from (i) a reducing agent and (ii) an amino acid selected from the group consisting of lysine and glycine and mixture thereof.

In a first embodiment, the product is then collected.

In a preferred embodiment of step a2), TNFα and KLH are firstly mixed together in the appropriate amounts, before adding glutaraldehyde.

Advantageously, TNFα and KLH are mixed at step a2) at a TNFα:KLH molar ratio ranging from 10:1 to 40:1. In some preferred embodiments, TNFα and KLH are mixed at step a) at a TNFα:KLH molar ratio ranging from 30:1 to 40:1. Preferably, TNFα and KLH are mixed at step a2) at a TNFα:KLH molar ratio ranging from 35:1 to 40:1.

In this variant method, the features related to the "quenching reaction after glutaraldehyde" as described in the main method hereabove, apply *mutatis mutandis.*

In this variant method, step b) is performed and the features related to step b), i.e. removal of compounds having a molecular weight of less than 10 kDa as described in the main method hereabove, apply *mutatis mutandis.*

In a second embodiment, after step b) and prior to collecting the product, formaldehyde is applied in a concentration/time of reaction condition ranging from at least 60 mM for at least 4 days, and then the reaction with formaldehyde is blocked by adding a quenching compound selected from (i) a reducing agent and (ii) an amino acid selected from the group consisting of lysine and glycine and mixture thereof. In this variant method, the features related to Step d): "quenching reaction after formaldehyde", as described in the main method hereabove, apply *mutatis mutandis.*

According to a preferred embodiment of the invention, just prior to collecting the product, a step of tangential flow filtration using a filtration membrane having a cut-off value of at least 100 kDa. (preferentially 300 kDa) is performed, resulting in that the substances having a molecular weight of less than 100 (preferably 300 kDa) are removed from the product. In this variant method, the features related to "removal of species of less than 100kDa, preferably of less than 300 kDa" as described in the main method hereabove, apply *mutatis mutandis.*

Optionally, the final immunogenic product according to the invention may be further processed for long term storage before use. In this variant method, the features related to lyophilization as described in the main method hereabove, apply *mutatis mutandis.*

**Brief description of the figures:**

**Figure 1:** (A) Percentage of cell viability in function of concentration of product according to test A. (B) Percentage of cytolytic activity in function of concentration of product according to test A.

**Figure 2:** (A) Comparison of EC50 of the tested products determined according to test A. (B) Comparison of Inactivation Factor of the tested products determined according to test A.

**Figure 3:** (A) Percentage of cell viability in function of concentration of product according to test B. (B) Percentage of cytolytic activity in function of concentration of product according to test B.

**Figure 4:** (A) Comparison of EC50 of the tested products determined according to test B. (B) Comparison of Inactivation Factor of the tested products determined according to test B.

**Figure 5:** Titers of anti-TNFa antibodies in mice immunized with the product of the invention.

**Figure 6:** Assessment of toxicity in mice (lethal shock model)

**Figure 7:** (A) SE-HPLC profiles of products of the invention after final filtration. (B) Evaluation of the presence of the product of the invention in the different fractions obtained after filtration.

**Figure 8:** (A) Immunogenicity of the filtered and non-filtered product of the invention. (B) Immunogenicity of the retentate (R) versus filtrate (F) of the filtrated product.

**Figure 9:** Development of arthritis in mice after administration of the vaccine of the invention.

**Figure 10:** Anti-TNFa antibody response in patients immunized with the vaccine of the invention.

**Figure 11:** State of clinical remission in patients immunized with the vaccine of the invention.

**Figure 12:** State of clinical remission in seropositive and seronegative patients immunized with the vaccine of the invention.

**Figure 13:** (A) Percentage of cell viability in function of concentration of product according to test A. (B) Percentage of cell viability in function of concentration of product according to test B.

**Figure 14 :** (A) Comparison of EC50 of the tested products determined according to test B. (B) Comparison of Inactivation Factor of the tested products determined according to test B.

**EXAMPLES**

**Example 1 : Preparation of the product of the invention**

KLH in its native form is a di-decamer structure (non covalent tubular assembly of 20 subunits) corresponding to a homopolymer of subunits KLH1 or KLH2 (KLH1:KLH2 $\cong$ 0.9:1); molecular Weight (MW) $\cong$ 8 $10^6$ Da. Native KLH also includes a consistent proportion of higher MW multimers and lower MW decamers. Keyhole Limpet Hemocyanin (KLH) was extracted from the lymph of the marine gastropod mollusk *Megathura crenulata* and then purified under GMP condition. Results from stability assays performed in storage conditions at a temperature of 2-8°C showed that the shelf life of the purified KLH is of 36 months at 2-8°C.

Recombinant human TNF-$\alpha$ was produced in *E. coli* under GMP conditions.

Batches of the product of the invention at 370 mg TNF scale were produced using the manufacturing process developed below.

14

### a) Conjugation with Glutaraldehyde

The TNFα is diluted in a buffer (130 mM di-sodium, hydrogen phosphate, 133 mM sodium Chloride and 6.6 mM EDTA, pH 7.8) to obtain a solution at 1.05 mg/mL and 1% of DMSO is added. After incubation at 22 ± 3°C during 30 min, a working buffer (100 mM di-solution, hydrogen phosphate, 150 mM Sodium Chloride and 5 mM EDTA pH 7.8) is added to dilute the TNF mixture to 0.51 mg/mL.

The filtered KLH is added to the TNF solution with a TNFα:KLH ratio of 1:0.58, (corresponding to a molar ratio of 1 monomer of KLH for 37 monomers of TNFα) based on UV concentration.

The conjugation is carried out with glutaraldehyde (added to reach 25 mM in the reaction medium), added from a stock solution of 2.5% w/v with a peristaltic pump and the solution is mixed during a defined time at 23 ± 2 °C.

### b) Quenching with glycine

The reaction is quenched with Glycine 0.1 M during 15 mins.

### c) First tangential flow filtration (TFF 1)

The first TFF is performed with a Pall Minim II TFF system and a polyethersulfone membrane of 0.02 $m^2$ with a molecular weight cut off of 10 kDa sanitized with 0.5 M NaOH and equilibrated with the working buffer.

The quenched solution is then clarified by 0.22 pm-filtration. The intermediate is diluted twice in working buffer and then diafiltered by tangential flow filtration (TFF) and 12 volumes of working buffer. The retentate is harvested and is stored for less than 20 hours.

### d) Inactivation with Formaldehyde

Formaldehyde is added to the retentate to reach a defined final concentration using a peristaltic pump. The inactivation reaction is performed during a defined time in an incubator set to 37±2°C with a daily mixing of the solution with a magnetic stirrer.

### e) Quenching with glycine

The reaction is then quenched with 0.38 M of Glycine during 1 hour.

### f) Second tangential filtration (TFF 2)

The second TFF is performed with a Pall Minim II TFF system and a polyethersulfone membrane of 0.02 $m^2$ with a molecular weight cut off of 300 kDa sanitized with 0.5 M NaOH and equilibrated with the formulation buffer.

The quenched solution is clarified by 0.2 μm filtration. The intermediate is concentrated to have a starting tangential volume of ≈ 900 mL and next filtrated by TFF with 12 volumes of formulation buffer (1.47 mM KH2PO4, 8.1 mM Na2HPO4, 2.68 mM KCl, 136.9 mM NaCl, pH 7.3) to eliminate the low molecular weight homopolymers of TNF and the non reactive reagents. The retentate is harvested and then diluted to a theoretical concentration of 300 μg/mL based on concentration determination by BCA and then 0.2μm-filtered to obtain the product of the invention.

### Example 2 : Description of the preparation conditions of several products of the invention and comparison with the product described in WO 2007/022813

**Table 1**

| product | Glutaraldehyde step | Quenching 1 | Formaldehyde step | Quenching 2 |
|---|---|---|---|---|
| **B1** | 45'<br>25 mM | 0 | 66 mM<br>6 days | Gly 1 h RT<br>100 mM |
| **B2** | 120'<br>25 mM | Gly 1 h RT<br>0.1 M | 66 mM<br>6 days | Gly 1 h RT<br>100 mM |

(continued)

| product | Glutaraldehyde step | Quenching 1 | Formaldehyde step | Quenching 2 |
|---------|---------------------|-------------|-------------------|-------------|
| **B3** | 240'<br>25 mM | Gly 1 h RT<br>0.1 M | 66 mM<br>6 days | Gly 1 h RT<br>100 mM |
| **B5** | 120'<br>25 mM | Gly 1 h RT<br>0.1 M | 250 mM<br>6 days | Gly 1 h RT<br>380 mM |
| **B80** | 120'<br>25 mM | Gly 1 h RT<br>0.1 M | 250 mM<br>14 days | Gly 1 h RT<br>380 mM |
| **B11** | 240'<br>25 mM | Gly 1 h RT<br>0.1 M | 250 mM<br>6 days | Gly 1 h RT<br>380 mM |
| **B14** | 240'<br>25 mM | Gly 1 h RT<br>0.1 M | 250 mM<br>14 days | Gly 1 h RT<br>380 mM |
| **B140** | 240'<br>25 mM | Gly 1 h RT<br>0.1 M | 460 mM<br>14 days | Gly 1 h RT<br>700 mM |
| **GTP0902** | 240'<br>25 mM | Gly 1 h RT<br>0.1 M | 250 mM<br>14 days | Gly 1 h RT<br>380 mM |

B1 corresponds to the product described in WO2007/022813.

GTP0902 was obtained by the process described in Example 1 at the conditions mentioned in Table 1 and wherein a second tangential filtration was performed at step f) with a cut-off of 300 kDa. GTP0902 is a GMP clinical batch.

### Example 3 : The products of the invention are strongly inactivated as shown by Test A

This test is used to determine the percentage of inactivation of human $TNF\alpha$ bioactivity in the product of the invention.

The test is based on the cytolysis of murine L929 cells induced by human $TNF\alpha$ in the presence of Actinomycin D. This test is carried out at T0, i.e. the product is in liquid form and stored at 4°C.

*Materials and Methods*

L929 mouse fibroblasts cells (Sigma n°85011425) were plated at 1.5 $10^4$/cm$^2$ in Culture Medium (DMEM (Cambrex BE12604F) supplemented 10% FBS (Sigma F7524), 2 mM glutamine (Sigma G7513), 100 U/ml penicillin/ streptomycin (Sigma P0781) and 1 mM Sodium Pyruvate (Sigma S8636)) and cultured for 2 days at 37°C 5% $CO_2$ to obtain subconfluent monolayer.

L929 cells were then harvested and plated in 96 well flat bottom culture plates at 2 $10^4$ cells/well in 100 $\mu$l of Plating Medium (DMEM F12 (Cambrex BE12719F) supplemented with 2% FBS, 2 mM glutamine, 100 U/ml penicillin/ streptomycin and 1 mM Sodium Pyruvate) and cultured for 21 +/- 1 h at 37°C, 5%$CO_2$.

A series of ten two-fold dilutions of the product of the invention was prepared from 120 $\mu$l of the product of the invention at 6400 ng/ml ($TNF\alpha$ equivalent) diluted in 60 $\mu$l of Assay Medium (HL1 (Cambrex US77201) supplemented with 2 mM glutamine, 100 U/ml penicillin/streptomycin and 1 mM Sodium Pyruvate).

The concentration unit used is $TNF\alpha$ equivalent concentration. $TNF\alpha$ equivalent concentration makes it possible to compare different batches, with the same TNF content, in cellular bioassay and *in vivo* in the TNF shock model. A concentration in $TNF\alpha$ equivalent is determined as following:

[$TNF\alpha$ equivalent concentration] = (quantity of $TNF\alpha$ at the beginning of the process)-10%. If a final step of filtration with a cut-off of 300 kDa has been carried out in the process for preparing the product of the invention, 75 % of $TNF\alpha$ is removed (as evidenced on a radioactive test in which $TNF\alpha$ was radio-labeled) and the concentration in $TNF\alpha$ equivalent is determined as following: [$TNF\alpha$ equivalent concentration] = [(quantity of $TNF\alpha$ at the beginning - 10%) - 75 %]. Of note, yield is consistent during manufacturing process.

A series of ten three-fold dilutions of the standard (human $TNF\alpha$ 6.24 mg/ml, Boehringer ingelheim 03030R1) was prepared from 120 $\mu$l of human $TNF\alpha$ at 8 ng/ml in 60 $\mu$l of Assay Medium. EC50 of TNF from Boehringer ranges from 10 to 500 pg/ml.

At the end of culture time of L929 cells, cells were subconfluent. The wells of the flat-bottom culture plates were then emptied of the culture medium and 50 μl of each dilution were transferred into the wells of the flat-bottom culture plate.

50 μl of Assay Medium supplemented with Actinomycin D at 2 μg/ml (Sigma A9415) were added to each well.

The L929 cells were then cultured for 20 +/- 1 h at 37°C 5% $CO_2$.

At the end of the culture, 20 μl/well of a solution of MTS/PMS (100μl MTS/5μlPMS; Promega G5430) were added and the plate was incubated for another 4h at 37°C 5% $CO_2$.

The plate was then read at 490 nm on a DYNEX spectrophotometer, MRXII.

The percentage of viability was calculated as following:

$$\% = 1 - [(OD_{product} - OD_{TNFstandard})/(OD_{cells} - OD_{TNFstandard})]$$

$OD_{product}$ stands for the optical density of well with the product of the invention.

$OD_{TNFstandard}$ stands for the optical density of well with the standard TNFα at 200 ng/ml.

$OD_{cells}$ stands for the optical density of control well with no standard nor product of the invention.

*Results*

The products B1, B2, B3, B5, B80, B11, B14, B140 and GTP0902 were produced according to the conditions mentioned in Table 1 and stored at 4°C in liquid form for less than 10 days before test A was performed.

They were tested in the L929 bioassay (Test A) as described in Materials and Methods.

The percentage of viability of L929 cells was determined and results are shown in **Figure 1A** and **Table 2.**

**Table 2: percentage of cell viability (test A)**

| Equivalent hTNF alpha cone. (ng/mL) | B1 | B2 | B3 | B5 | B80 | B11 | B14 | B140 | Equivalent hTNF alpha conc. (ng/mL) | GTP0902 |
|---|---|---|---|---|---|---|---|---|---|---|
| 3200 | 2 | 4 | 14 | 35 | 76 | 81 | 101 | 106 | 9210 | 91 |
| 1600 | 5 | 8 | 33 | 60 | 83 | 87 | 100 | 98 | 3070 | 95 |
| 800 | 10 | 21 | 54 | 81 | 92 | 97 | 106 | 104 | 1023,333 | 104 |
| 400 | 19 | 48 | 73 | 88 | 94 | 99 | 104 | 101 | 341,1111 | 107 |
| 200 | 48 | 64 | 91 | 99 | 97 | 98 | 106 | 106 | 113,7037 | 107 |
| 100 | 69 | 92 | 103 | 102 | 98 | 105 | 106 | 104 | | |
| 50 | 81 | 101 | 100 | 103 | 100 | 100 | 104 | 105 | | |
| 25 | 100 | 105 | 95 | 97 | 102 | 97 | 108 | 103 | | |
| 12,5 | 105 | 95 | 105 | 98 | 103 | 100 | 108 | 106 | | |
| 6,25 | 111 | 114 | 108 | 107 | 101 | 99 | 108 | 104 | | |

**Figure 1A** shows the percentage of cell viability in function of increasing concentrations of the products. At the 100 ng/ml concentration, B1 (the product of WO2007/022813) killed 31 % of the cells, whereas the products of the invention killed less than 10% of the cells. In particular, B14 and GTP0902 killed less than 5% of cells at 100 ng/ml.

**Figure 1B** shows that at a concentration of 100 ng/ml of product, less than 40% of cells survived in the presence of B1 (the product of WO2007/022813), whereas more than 60% of cells survived in the presence of the products of the invention. In particular, almost 90% of cells survived in the presence of 100 ng/ml of B14 and GTP0902.

In conclusion, the products of the invention are strongly inactivated as shown by Test A.

Results were also analyzed as EC50, which is the concentration of the product necessary to reduce cell growth by 50%.

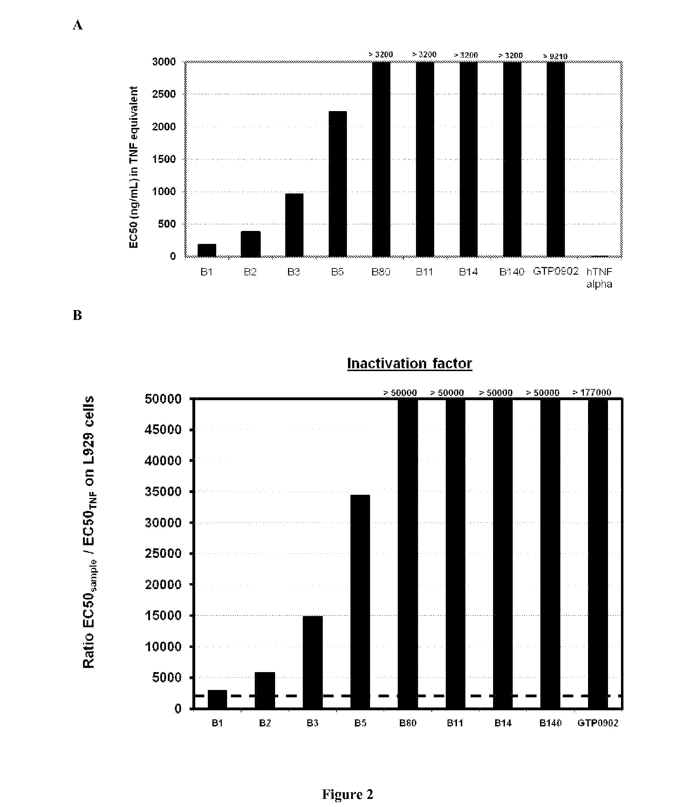

**Figure 2A and Table 3** shows that EC50 of B1 (the product of WO2007/022813) is extremely low (less than 200 ng/ml) compared to EC50 of the products of the invention.

**Table 3**

|  | B1 | B2 | B3 | B5 | B80 | B11 | B14 | B140 | GTP0902 | hTNF alpha |
|---|---|---|---|---|---|---|---|---|---|---|
| **EC50 (ng/ml)** | 189 | 378 | 965 | 2233 | >3200 | >3200 | >3200 | >3200 | >9210 | 0,0648 |

The Inactivation Factor of each product was calculated as following: EC50 product/$^{EC50}$TNF$\alpha$.

**Figure 2B and Table 4** shows that B1 (the product of WO2007/022813) has an extremely low Inactivation Factor (less than 4000) compared to the ones of the products of the invention (more than 10 000).

These results show that the products of the invention are at least 2.5x more inactive than B1 (the product of WO2007/022813). In particular, B14 and GTP0902 are more than 10 000x more inactive than B1.

**Table 4**

|  | B1 | B2 | B3 | B5 | B80 | B11 | B14 | B140 | GTP0902 |
|---|---|---|---|---|---|---|---|---|---|
| **EC50$_{sample}$ / EC50$_{TNF}$** | 2922 | 5824 | 14886 | 34444 | > 50000 | > 50000 | > 50000 | > 50000 | > 177000 |

## Example 4 : The products of the invention remains inactivated as shown by Test B

This test is used to measure the extent of reversion (regeneration of TNF$\alpha$ activity) when the products are stored in liquid form at 37°C for 6 weeks after production as classically done for inactivated vaccine. This test is performed to make sure the inactivation of the product of the invention remains stable during time or after administration.

*Materials and Methods*

L929 mouse fibroblasts cells (Sigma n°85011425) were plated at 1.5 10$^4$/cm$^2$ in Culture Medium (DMEM (Cambrex BE12604F) supplemented 10% FBS (Sigma F7524), 2 mM glutamine (Sigma G7513), 100 U/ml penicillin/ streptomycin (Sigma P0781) and 1 mM Sodium Pyruvate (Sigma S8636)) and cultured for 2 days at 37°C 5% CO$_2$ to obtain subconfluent monolayer.

L929 cells were then harvested and plated in 96 well flat bottom culture plates at 2 10$^4$ cells/well in 100 $\mu$l of Plating Medium (DMEM F12 (Cambrex BE12719F) supplemented with 2% FBS, 2 mM glutamine, 100 U/ml penicillin/ streptomycin and 1 mM Sodium Pyruvate) and cultured for 21 +/- 1 h at 37°C, 5%CO$_2$.

A series of five three-fold dilutions of the product of the invention was prepared from 120 $\mu$l of the product of the invention at 6400 ng/ml (TNF$\alpha$ equivalent) diluted in 60 $\mu$l of Assay Medium (HL1 (Cambrex US77201) supplemented with 2 mM glutamine, 100 U/ml penicillin/streptomycin and 1 mM Sodium Pyruvate).

The concentration unit used is TNF$\alpha$ equivalent concentration (Example 4) or total proteins determined using a BCA test (Example 12). TNF$\alpha$ equivalent concentration makes it possible to compare different batches, with the same TNF content, in cellular bioassay and *in vivo* in the TNF shock model. A concentration in TNF$\alpha$ equivalent is determined as following:

[TNF$\alpha$ equivalent concentration] = (quantity of TNF$\alpha$ at the beginning of the process)-10%.

If a final step of filtration with a cut-off of 300 kDa has been carried out in the process for preparing the product of the invention, 75 % of TNF$\alpha$ is removed (as evidenced on a radioactive test in which TNF$\alpha$ was radio-labeled) and the concentration in TNF$\alpha$ equivalent is determined as following: [TNF$\alpha$ equivalent concentration] = [(quantity of TNF$\alpha$ at the beginning - 10%) - 75 %]. Of note, yield is consistent during manufacturing process.

A series of ten three-fold dilutions of the standard (human TNF$\alpha$ 6.24 mg/ml, Boehringer Ingelheim 03030R1) was prepared from 120 $\mu$l of human TNF$\alpha$ at 8 ng/ml in 60 $\mu$l of Assay Medium. EC50 of TNF from Boehringer ranges from 10 to 500 pg/ml.

At the end of culture time of L929 cells, cells were subconfluent. The wells of the flat-bottom culture plates were then emptied of the culture medium and 50 $\mu$l of each dilution were transferred into the wells of the flat-bottom culture plate.

50 $\mu$l of Assay Medium supplemented with Actinomycin D at 2 $\mu$g/ml (Sigma A9415) were added to each well.

The L929 cells were then cultured for 20 +/- 1 h at 37°C 5% CO$_2$.

At the end of the culture, 20 $\mu$l/well of a solution of MTS/PMS (100$\mu$l MTS/5$\mu$lPMS; Promega G5430) were added and the plate was incubated for another 4h at 37°C 5% CO$_2$.

The plate was then read at 490 nm on a DYNEX spectrophotometer, MRXII.

The percentage of viability was calculated as mentioned in Example 3.

*Results*

The products B1, B2, B3, B5, B80, B11, B14, B140 and GTP0902 were produced according to the conditions mentioned in Table 1 and stored at 37°C in liquid form during 6 weeks before test B was performed.

They were tested in the L929 bioassay (Test B) as described in Materials and Methods.

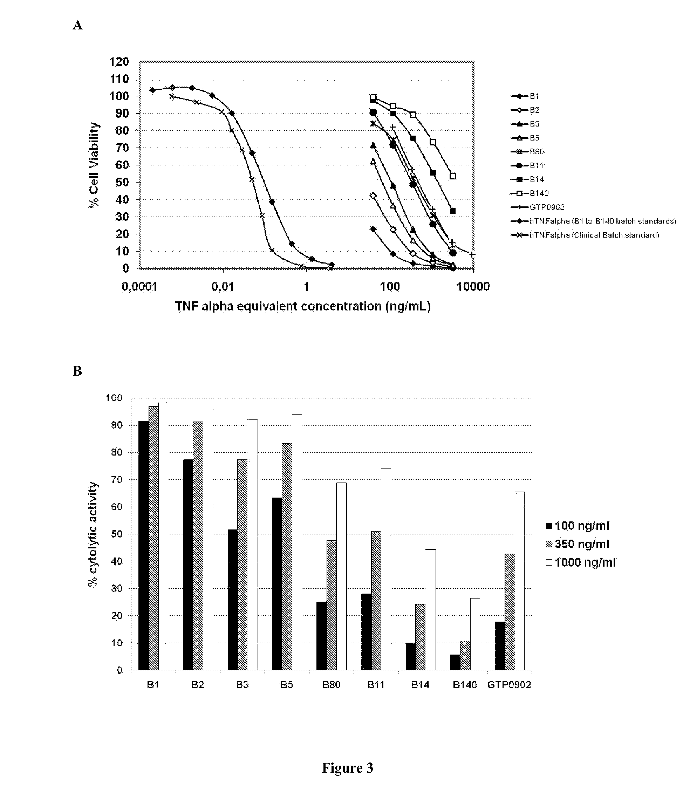

The percentage of viability of L929 cells was determined and results are shown in **Figure 3A.**

**Figure 3A** and **Table 5** shows the percentage of cell viability in function of increasing concentrations of the products. At the 100 ng/ml concentration, B1 (the product of WO2007/022813) killed more than 90% of the cells, whereas the products of the invention killed less than 65% of the cells. In particular, B14 and GTP0902 killed about 20% of cells at 100 ng/ml.

**Table 5: percentage of cell viability (test B)**

| Equivalent hTNF alpha conc. (ng/mL) | B1 | B2 | B3 | B5 | B80 | B11 | B14 | B140 | Equivalent hTNF alpha conc. (ng/mL) | GTP0902 |
|---|---|---|---|---|---|---|---|---|---|---|
| 3200 | 0 | 1 | 2 | 2 | 14 | 9 | 33 | 54 | 9210 | 8 |
| 1066,7 | 1 | 4 | 8 | 6 | 31 | 26 | 56 | 74 | 3070 | 15 |
| 355,6 | 3 | 9 | 23 | 17 | 52 | 49 | 76 | 89 | 1023,333 | 34 |
| 118,5 | 9 | 23 | 48 | 37 | 75 | 72 | 90 | 94 | 341,1111 | 57 |
| 39,5 | 23 | 42 | 72 | 62 | 84 | 91 | 98 | 99 | 113,7037 | 82 |

**Figure 3B** shows that at a concentration of 100 ng/ml of product, less than 10% of cells survived in the presence of B1 (the product of WO2007/022813), whereas more than 35% of cells survived in the presence of the products of the invention. In particular, more than 80% of cells survived in the presence of 100 ng/ml of B14 and GTP0902.

In conclusion, the products of the invention remain strongly inactivated as shown by Test B.

Results were also analyzed as EC50, which is the concentration of the product necessary to reduce cell growth by 50%.

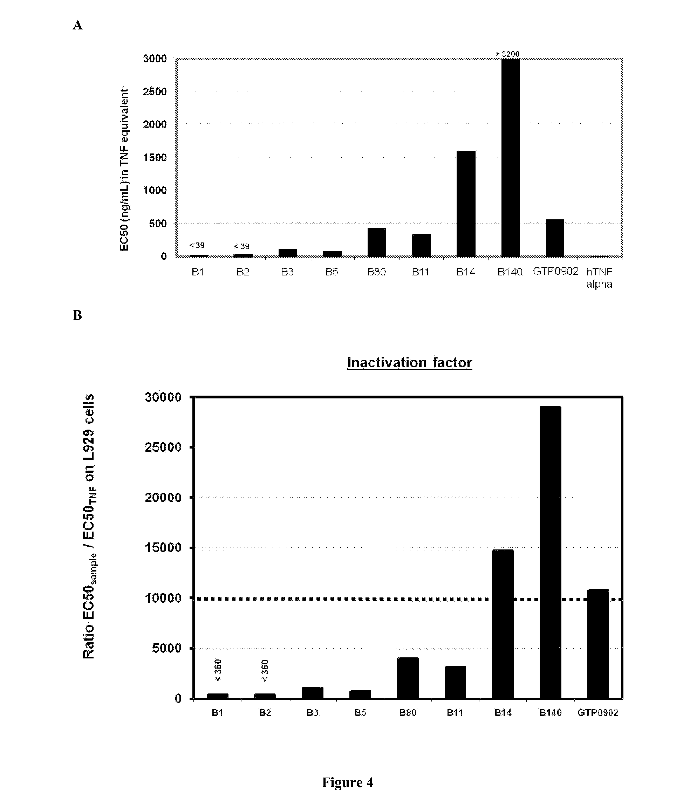

**Figure 4A** and Table 6 shows that EC50 of B1 (the product of WO2007/022813) is extremely low (less than 50 ng/ml) compared to EC50 of the products of the invention.

**Table 6**

| | B1 | B2 | B3 | B5 | B80 | B11 | B14 | B140 | GTP0902 | hTNF alpha |
|---|---|---|---|---|---|---|---|---|---|---|
| **EC50** | <39.5 | <39.5 | 113 | 77 | 433 | 344 | 1604 | >3200 | 559 | 0,109 |

The Inactivation Factor of each product was calculated as following: $EC50_{product}/EC50_{TNF\alpha}$.

**Figure 4B** and Table 7 shows that B1 (the product of WO2007/022813) has an extremely low Inactivation Factor (less than 500) compared to the ones of the products of the invention.

These results show that the products of the invention remain at least 3x more inactive than B1 (the product of WO2007/022813). In particular, B14 and GTP0902 remain more than 30x more inactive than B1.

**Table 7**

| | B1 | B2 | B3 | B5 | B80 | B11 | B14 | B140 | GTP0902 |
|---|---|---|---|---|---|---|---|---|---|
| **$EC50_{Sample}/EC50_{TNF}$** | < 360 | < 360 | 1030 | 709 | 3968 | 3151 | 14683 | > 29000 | 10728 |

**Example 5 : Determination of the presence of free TNFα homopolymers in the product of the invention (Test C)**

Homopolymers of TNFα and of KLH were purified after selective depletion by an immunocapture step using magnetic beads coated with anti-TNFα monoclonal antibodies (step 1) or with anti-KLH polyclonal antibodies (step 1). By using anti-TNFα antibodies coated beads, free TNFα homopolymers and the product of the invention were depleted from the supernatant, allowing quantification of free KLH homopolymers by specific ELISA (step 2). In the same manner, by using anti-KLH antibodies coated beads, free KLH homopolymers and the product of the invention were depleted from the supernatant, allowing quantification of free TNFα homopolymers by specific ELISA (step 2).

The quantitative determination of free TNF$\alpha$ homopolymers and free KLH homopolymers in the product of the invention was conducted using 2 specific ELISA method-based tests (respectively TNF-TNF and KLH-KLH ELISA). In addition a KLH-TNF ELISA was carried out on the supernatant to control the complete depletion of the product of the invention from the test samples.

Principle of the immunocapture with magnetic beads coated with anti-KLH Abs.

With immunocapture using beads coated with anti- KLH Antibody, homopolymers of TNF$\alpha$ can be quantified in the supernatant using "TNF-TNF" ELISA. Complete depletion of heteropolymers and homopolymers of modified TNF$\alpha$ in the supernatant is showed by an absence of signal using "KLH-KLH" and "KLH-TNF" ELISA.

_Materials and Methods_

_Preparation of Beads coupled with anti-KLH or_ $\alpha$_nti- TNF$\alpha$ antibodies_ 1.3 10$^9$ beads (Dynabeads® M270 Epoxy, Invitrogen 14302D) were diluted in PBS to reach 20 mg/ml final concentration and incubated for 10 min.

After washing by using the magnet, the beads were resuspended in 486 $\mu$l of Borate Buffer 100 mM pH 9.

333 $\mu$l of Ammonium sulphate 3M were added to reach final concentration at 1M.

182 $\mu$l of monoclonal antibody anti-TNF$\alpha$ (3B2/1H4/2E5-SO8038b 2.2 mg/ml) or 235 $\mu$l of polyclonal anti KLH (S030.07122.1 1.7 mg/ml) were then added and the mixture incubated during 12-16h at 37°C. The beads were then harvested using the magnet.

1 ml of PBS 2% BSA was used to resuspend the beads for blocking the reaction and unspecific site then the mixture was incubated during 12-16h at 4°C. The beads were then harvested using the magnet.

_Incubation with test samples_

Coated and non-coated beads (20 mg/ml) were mixed with the sample to be tested (product diluted at 1 $\mu$g/ml in PBS 1% BSA) and then incubated during 12-16h at 4°C. The supernatant was then harvested using the magnet and analyzed by ELISA.

_KLH-KLH ELISA_

The sandwich KLH-KLH ELISA was carried out as well known in the art. The capture antibody (rabbit polyclonal antibody anti-KLH affinity purified (600-401-466, Rockland, 1 mg/ml)) was coated at 100 ng/well. The primary antibody (biotinylated rabbit polyclonal antibody anti-KLH affinity purified (600-406-466, Rockland, 1 mg/ml)) was used at 25 ng/ml.

The quantification of homopolymers of KLH in the sample was determined using a modified KLH as standard. The standard concentrations (from 400 to 15.625 ng/mL) were prepared by serial two-fold dilutions in Dilution Buffer

A Poly-Streptavidin-HRP (1/5000) is used to detect the reaction and the complex is developed by o-phenylenediamine dihydrochloride (OPD) substrate solution. After stopping the enzymatic reaction, the intensity of the resulting color is determined by spectrophotometric methods at 490 nm (reference filter at 650 nm).

_TNF-TNF ELISA_

The sandwich TNF-TNF ELISA was carried out as well known in the art. The capture antibody (goat polyclonal anti- hu TNF$\alpha$ affinity purified (R&D system, AF210NA, 1 mg/ml)) was coated at 100 ng/well. The primary antibody (biotinylated goat polyclonal anti-hu TNF$\alpha$ affinity purified (R&D system, BAF210, 0.5 mg/ml)) was used at 75 ng/ml. The quantification of homopolymers of TNF in the sample was determined using a modified TNF as standard. The standard concentrations (from 100 to 0.391 ng/mL) were prepared by serial two-fold dilutions

A Poly-Streptavidin-HRP (1/5000) is used to detect the reaction and the complex is developed by o-phenylenediamine dihydrochloride (OPD) substrate solution. After stopping the enzymatic reaction, the intensity of the resulting color is determined by spectrophotometric methods at 490 nm (reference filter at 650 nm).

_KLH TNF ELISA_

The sandwich KLH-TNF ELISA was carried out as well known in the art. The capture antibody (rabbit polyclonal antibody anti-KLH affinity purified (600-401-466, Rockland, 1 mg/ml)) was coated at 100 ng/well. The primary antibody (biotinylated goat polyclonal anti-hu TNF$\alpha$ affinity purified (R&D system, BAF210, 0.5 mg/ml)) was used at 75 ng/ml.

A Poly-Streptavidin-HRP (1/5000) is used to detect the reaction and the complex is developed by o-phenylenediamine dihydrochloride (OPD) substrate solution. After stopping the enzymatic reaction, the intensity of the resulting color is determined by spectrophotometric methods at 490 nm (reference filter at 650 nm).

*Method for determining percentage of free TNFα or KLH homopolymers*

| Capture supernatant identification | TNFα concentration | KLH concentration | KLH-TNF Control |
|---|---|---|---|
| Anti-TNFα coated beads | C = determined using TNF-TNF ELISA | F = determined using KLH-KLH ELISA | G = control of TNF depletion evaluated by KLH-TNF ELISA |
| Anti-KLH coated beads | E = determined using TNF-TNF ELISA | D = determined using KLH-KLH ELISA | H = control of KLH depletion evaluated by KLH-TNF ELISA |
| Non-coated beads | A = determined using TNF-TNF ELISA | B = determined using KLH-KLH ELISA | I = control evaluated by KLH-TNF ELISA |

The TNFα concentrations A, C, E are determined by comparing the optical density to optical densities of a series of dilutions of TNFα carried out on the same plate.

The KLH concentrations B, D, F are determined by comparing the optical density to optical densities of a series of dilutions of KLH carried out on the same plate.

Controls (G,H,I) are determined by comparing the optical densities without immunocapture (I) and after immunocapture with anti-TNFα antibodies (G) and after immunocapture with anti-KLH antibodies.

E corresponds to free TNFα homopolymers.

F corresponds to free KLH homopolymers.

A corresponds to TNFα-KLH polymers + free TNFα homopolymers

B correspond to TNFα-KLH polymers + free KLH homopolymers. C and G are used as control of depletion to confirm the complete depletion of TNFα-KLH polymers + free TNFα homopolymers using immunocapture using anti-TNFα antibodies. D and H are used as control of depletion to confirm the complete depletion of TNFα-KLH polymers + free KLH homopolymers using immunocapture using anti-KLH antibodies

*Results*

The product GTP0902 and other clinical batches were tested for the presence of free TNFα homopolymers.

The KLH-KLH ELISA carried out on supernatant obtained from the sample to be tested mixed with the anti-TNFα coated beads showed that there is no free KLH homopolymers in the product GTP0902.

Consequently, the percentage of free TNFα homopolymers was calculated as E/A* 100. Results are shown in Table 8.

**Table 8**

| | Free homopolymers of TNF (%) | Free homopolymers of KLH (%) |
|---|---|---|
| **07111DO** | 14 | 0 |
| **07271DO** | 21 | 0 |
| **07421NX** | 25 | 0 |
| **2020339** | 16 | 0 |
| **GTP0902** | 15 | 0 |
| **DTP0903** | 13 | 0 |
| **GTP1003** | 12 | 0 |
| **Average** | **17** | **0** |

Consequently, the results show that the products of the invention, which have been obtained according to a method performing a final step of filtration with a cut-off of 300 kDa, contain no free KLH homopolymers and less than 30 % of free TNFα homopolymers.

## Example 5 : Immunogenicity of the products of the invention

*Materials and Methods*

Two groups of Balb/c were immunized with 1 μg (TNFα equivalent) of B2, B3, B5, B80, B14, B140 or B1 (the product of WO2007/022813) emulsified in ISA-51. Immunizations were done at days 0 and 21 with a 1-week delay between the two groups. At day 28, sera were collected and tested for the presence of anti-huTNF-α antibodies by ELISA.

*Results*

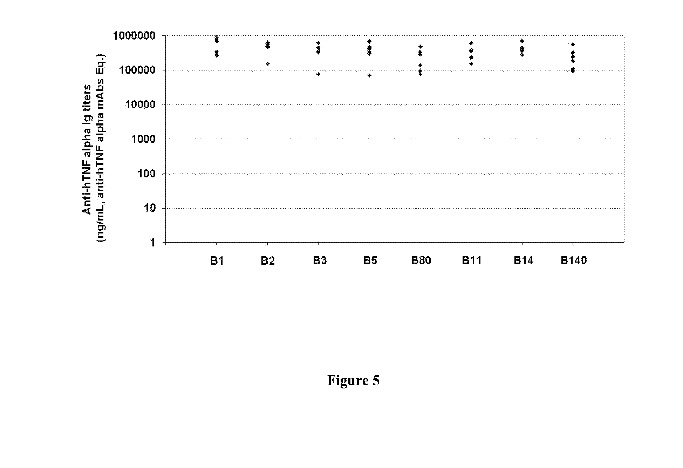

As shown in **Figure 5,** all products led to high levels of anti-TNFα titers.
In conclusion, the products of the invention have the same immunogenicity property than the product B 1.

## Example 6 : Toxicity of the products of the invention

The product toxicity was evaluated in a TNFα-mediated shock assay.

*Materials and Methods*

This assay is described in Lehmann et al. JEM 1987, 165: 657-663.
Briefly, mice were intraperitoneally injected with 100 μl of a solution comprising 20 mg of D-galactosamine and 11 μg of TNFα (control - stored at 4°C) or 11 μg (TNFα equivalent) of the products B1, B80, B14, B140 that have been stored in liquid form at 37°C for 6 weeks. Mortality was assessed after 24h.

*Results*

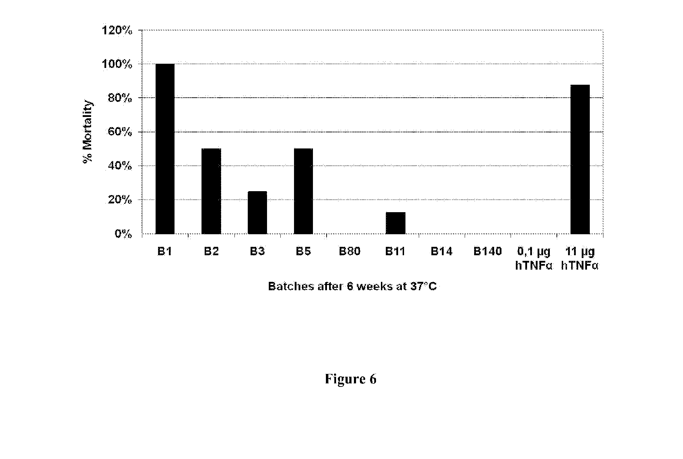

As shown in **Figure 6,** the product B1 (product of WO2007/022813) is as lethal as TNFα.
On the contrary, the products of the invention were not toxic.
According to the information provided by the EPAR of Beromun®, the Maximal Tolerated Dose (MTD) is 150-200 μg/m$^2$. Based on an average body surface of 1.9 m$^2$, the MTD of TNF corresponds to 285 μg.
An administered dose of the product of the invention represents 180 μg of proteins. In the quality control and stability results on the different produced batches, the level of inactivation after reversion was always above 10,000 fold (4 log) compared to endogeneous TNF. Therefore, the TNF activity administrated in a clinical dose (180 μg) is less than 18 ng, which is 15,000 times lower than the MTD of TNF providing an important safety margin (15833). The TNF activity administrated in a clinical dose (360 μg) corresponds to less than 36 ng, which is 7,500 times lower than the MTD of TNF providing an important safety margin.

## Example 7: Immunogenicity of the product of the invention when a step of filtration at the end of the process is performed

The product of the invention was produced according to the method described in Example 1 in the conditions of GTP0902, except that the TNFα used was labeled with I*125.
A tangential flow filtration was carried out with different cut-off at the end of the production process of the products of the invention (step f).

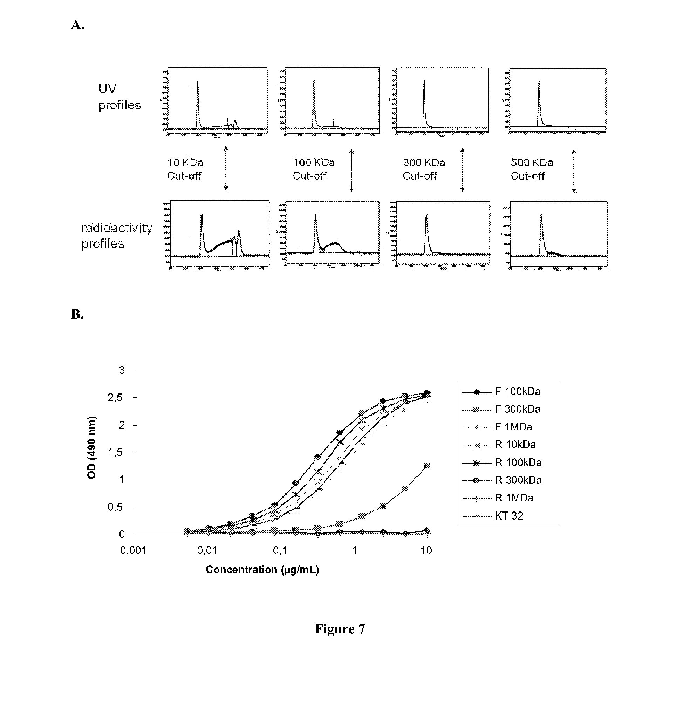

**Figure 7** shows the SE-HPLC profiles of I* 125 labeled product after final filtration of 10 kDa, 100 kDa, 300 kDa or 500 kDa.
A TNF-KLH ELISA was carried out on the different fractions obtained after filtration according to the method described in Example 5.

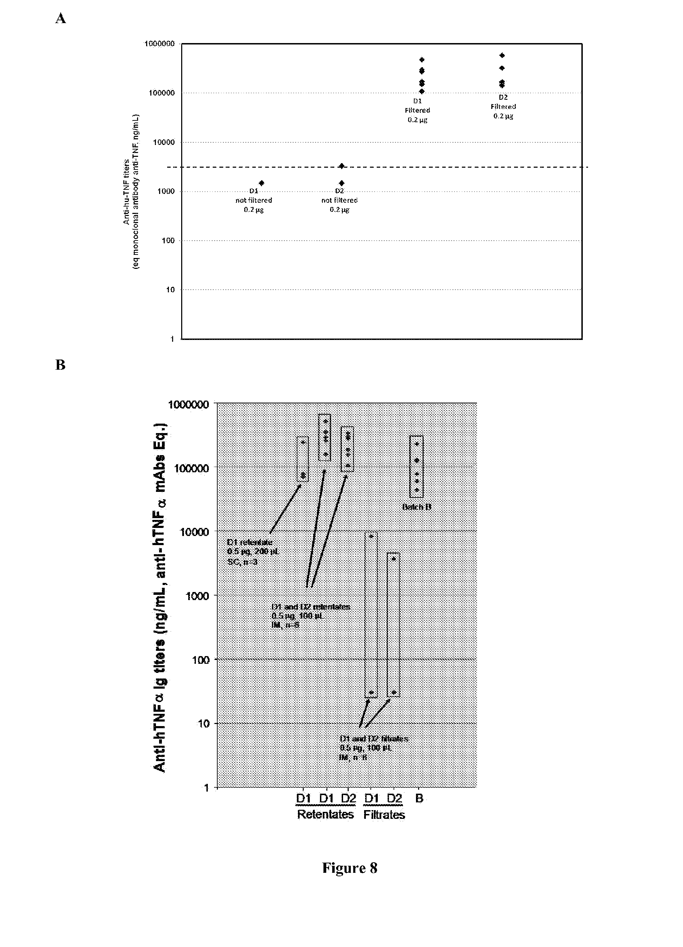

**Figure 8A** shows that the product of the invention is not present in the filtrate 100 kDa and begins to be detectable in the filtrate 300 kDa.
Immunogenicity of the product filtrated or not with a cut-off of 300 kDa was assessed by immunization of mice with 0.2 μg or 0.5 μg of product filtered or not filtered according to the method described in Example 5.
**Figure 8A** shows that the filtered product (two batches D 1 and D2) led to higher levels of anti-TNFα titers.
**Figure 8B** corresponds to the assessment of immunogenicity of retentates versus filtrates and shows that the 300 kDa filtrates are non immunogenic.

**Example 8 : Examples of compositions comprising the product of the invention**

Two illustrative compositions are described in Tables 9 and 10.

**Table 9**

| Composition 1 | |
| --- | --- |
| Components | Quantity |
| Product of the invention | 180 µg |
| Potassium dihydrogen phosphate | 140 µg |
| Disodium dihydrogen phosphate | 805 µg |
| Potassium chloride | 140 µg |
| Sodium chloride | 5600 µg |
| Mannitol | 30 mg |

**Table 10**

| Composition 2 | |
| --- | --- |
| Components | Quantity |
| Product of the invention | 220 µg |
| Potassium dihydrogen phosphate | 171 µg |
| Disodium dihydrogen phosphate | 984 µg |
| Potassium chloride | 171 µg |
| Sodium chloride | 6844 µg |
| Mannitol | 30 mg |

**Example 9 : Example of a vaccine comprising the product of the invention**

An example of vaccine according to the invention is described in Table 11.

**Table 11**

| Emulsion | |
| --- | --- |
| Components | Quantity |
| Product of the invention | 180 µg |
| Potassium dihydrogen phosphate | 140 µg |
| Disodium dihydrogen phosphate | 805 µg |
| Potasssium chloride | 140 µg |
| Sodium chloride | 5600 µg |
| Drakeol 6VR (mineral oil) | 0.22 mg |
| Montanide 80 (mannide monooleate) | 0.03 mg |
| Mannitol | 30 mg |
| Water for injection | 0.3 ml |
| Total volume | 0.6 ml |

**Example 10 : Treatment of arthritis in hTNFα transgenic mice**

Example 10 discloses the effectiveness of the product of the invention for treating a disease linked to an over-production of TNFα in a non-human mammal.

Briefly, a first group of 10 hTNFα transgenic mice described by Hayward et al. (2007, BMC Physiology, Vol. 7 : 13-29) were intramuscularly injected with a vaccine composition consisting of an emulsion of a human TNFα kinoid in ISA 51 that was prepared as described in Example 9. An amount of vaccine composition containing 4 µg of human TNFα kinoid has been administered i.m. at Day 0 (D0), Day 7 (D7) and Day 28 (D28), respectively. A second group of 10 transgenic mice were intramuscularly injected with a volume of Phosphate Buffered Saline (PBS) identical to the volume of vaccine composition injected to the first group of transgenic mice.

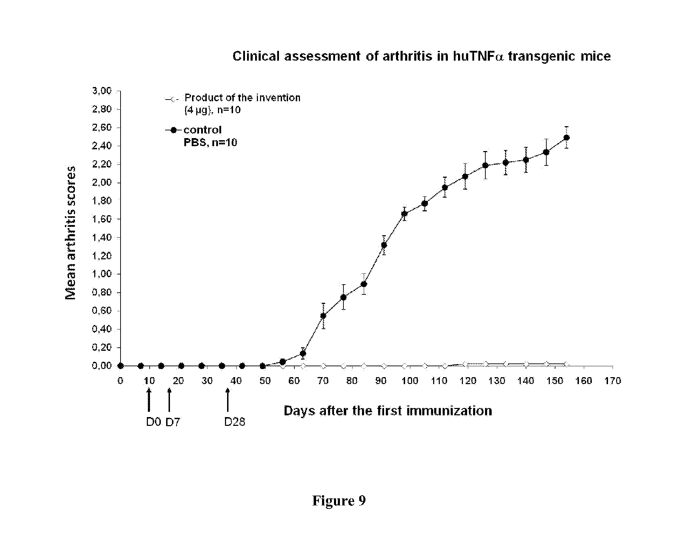

The mean arthritis scores were measured in the two groups of mice and the results are shown in **Figure 9.** The mean arthritis scores were measured as described by Lee et al. (2009, J Pharmacol Sci, Vol. 109 : 211-221).

The results show that arthritis rapidly developed in mice administered with PBS, whereas arthritis was completely blocked in the animals which have received the invention's vaccine composition.

**Example 11 : Treatment of Crohn's Disease**

Example 11 discloses the protocol of a phase I/II, open-label, escalating dose, "optimal two-stage", study of immunization in Crohn's Disease patients of the product of the invention.

*A. Clinical study protocol*

1. Indication/Study population

Patients with Crohn's disease and aged between 18 and 65 years old were immunized with three doses of the vaccine of the invention according to Example 9.

2. Rationale

This study is designed to assess the safety, reactogenicity, and immunogenicity of the candidate product of the invention combined with ISA-51 adjuvant in patients with Crohn's disease. The safety profile and the immune response to three doses of these candidate kinoids was evaluated at three dosages (60, 180, and 360 µg) administered on Days 0, 7, and 28.

3. Study design

Phase I/II, "optimal two-stage", multicentre, international, non-randomized study with three groups:

- Group A: 3 subjects receiving the vaccine of the invention (60 µg of the product of the invention) combined with adjuvant ISA51,

- Group B: 9 subjects receiving the vaccine of the invention (180 µg of the product of the invention) combined with adjuvant ISA51,

- Group C: 9 subjects receiving the vaccine of the invention (360 µg of the product of the invention) combined with adjuvant ISA51.

4. Duration of the study

All subjects with a positive anti-TNFα antibody response (defined as a 3-fold increase with respect to baseline) will be followed up for safety until normalization of antibody titers or at least until Day 140. Subjects with no antibody response will be followed until Day 140.

*B. Results of the clinical study*

Firstly, the results of the clinical study have shown that none of the patients treated with the vaccine of the invention have experienced serious adverse effects, which results fully confirm that the TNFα biological activity has been stably and irreversibly inactivated.

Secondly, it is underlined that none of the patients initially selected have been withdrawn during the course of the clinical study. Notably, none of the initially selected patients have been affected with an unexpected infection (one case of sinusitis was reported).

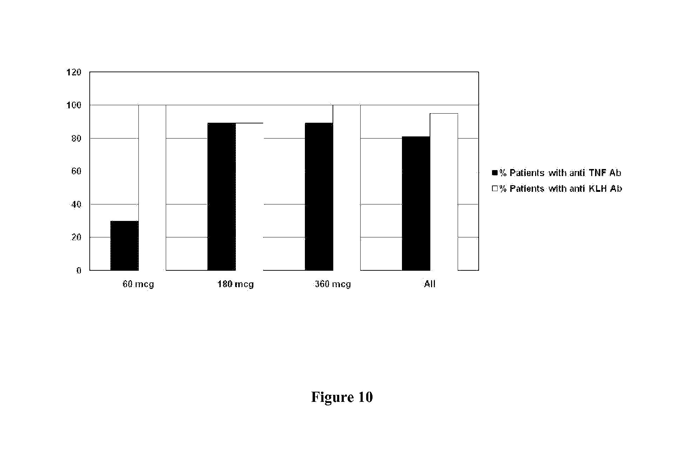

Further, as it is shown in **Figure 10,** an anti-TNFα antibody response has been measured in almost all patients: 33% of patients at 60 μg, and 89% of patients both at 180 μg and 360 μg.

As shown in **Figure 10,** the vaccine of the invention is rapidly therapeutically effective in Crohn's disease patients, since at the lower dosage of 60 μg, more than 65% of the patients exhibited a reduction of the CDAI score by more than 70% at Week 4 after immunization (CDAI-Crohn's Disease Activity Index- score values measured as described by Naber et al., The Journal of Medicine, Vol. 61 (n° 4) : 105-110).

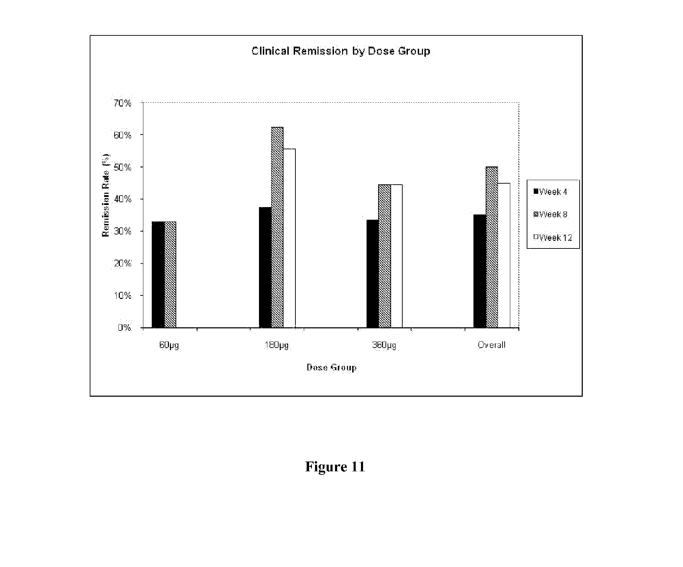

Further, the results depicted in **Figure 11** show that the administration of the vaccine of the invention to Crohn's disease patients induces a state of clinical remission in most of the patients. More precisely, it is shown in **Figure 11** that, for the lowest dosage of 60 μg, more than 30% of the patients exhibit a CDAI score of less than 150 at Week 4 and 8 after injection. Moreover, **Figure 11** shows that, at the dosage of 180 μg, and at Week 8 after injection, 67% of the patients exhibit a CDAI score value of less than 150.

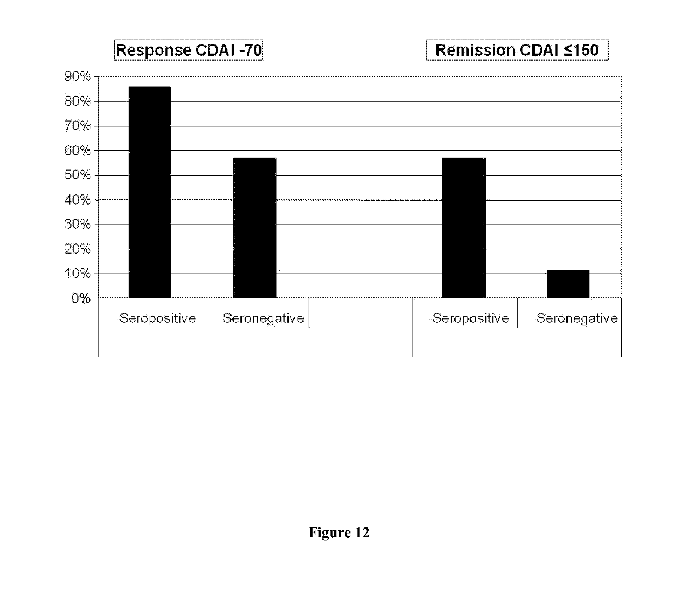

It is also shown in **Figure 12** that more than 85% of the anti-TNFα seropositive patients have experienced a therapeutic benefit, with a reduction of the CDAI score value of more than 70 points. It is also underlined that remission (CDAI score value equal or less than 150) was observed in more than 55% of the anti-TNFα seropositive patients, whereas remission was seen in only about 10% of the anti-TNFα seronegative patients.

As it is shown in Table 12 below, the high therapeutic effectiveness of the vaccine of the invention is illustrated by a high percentage of patients experiencing a Crohn's disease remission, as compared with the well known therapeutic anti-TNFα monoclonal antibodies Infliximab, Adalimumab and Certolizumab.

**Table 12**

| Product | Evaluation Time points | Remission (ITT-like) |
|---|---|---|
| Product of the invention | Week 4 | 35% |
| | Week 8 | 50% |
| | Week 12 | 45% |
| Infliximab | Week 12 (Targan 1997) | 24% |
| | Week 30 (Rutgeerts 2004) | 24% |
| Adalimumab | Week 4 (Hanauer 2006) | 36% |
| | Week 26 (Colombe 2007) | 23% |
| Certolizumab | Week 26 (Schreiber 2007) | 31% |

Targan 1997, NEJM, 340:1029-35

Rutgeerts 2004, Gastroenterology 126:402

Hanauer 2006, Gastroenterology 130(6):1929-30

Colombe 2007, Am Journ Gastroenterol. 102(sup2):5496-7

Schreiber 2007, NEJM 357(13)1357

Example 12 : The products of the invention are strongly inactivated as shown by Test A and remains inactivated as shown by Test B.

3 batches (808, 901, 903) were obtained by the method described in Example 1, wherein step a) is performed for 240 min at 25 mM final concentration of Glutarldehyde, step c) is performed for 14 days at 250 mM final concentration of Formaldehyde and a filtration with a cut-off of 300 kDa is performed in step f).

The products are stored in liquid form at 4°C or for 6 weeks at 37°C.

Test A was performed according to Example 3.

The following results are expressed in concentration of total proteins, as determined by the BCA protein assay.

The BCA protein assay is a detergent-compatible formulation based on bicinchoninic acid (BCA) for the colorimetric detection and quantitation of total protein. This method combines the well-known reduction of $Cu^{2+}$ to $Cu^{1+}$ by protein in alkaline medium (the biuret reaction) with the highly sensitive and selective colorimetric detection of the cuprous cation ($Cu^{1+}$) using a unique reagent containing bicinchoninic acid. The purple-coloured reaction product of this assay is formed by the chelation of two molecules of BCA with one cuprous ion. This water-soluble complex exhibits a strong absorbance at 562 nm that is linear with increasing protein concentrations over a broad working range of 20-2000 μg/ml.

It was then determined that 60 µg of total proteins correspond to 25 µg of TNFα equivalent.

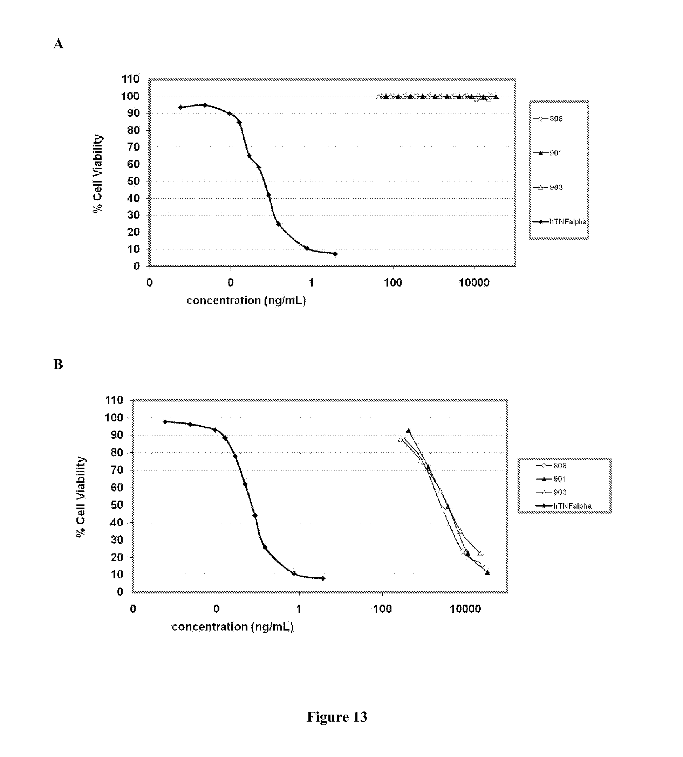

Results are shown in **Figure 13A** and Table 13.

**Table 13: percentage of cell viability (test A)**

| Conc (ng/ml) | 808 | Conc (ng/ml) | 901 | Conc (ng/ml) | 0903 | conc. (ng/ml) | hTNFalpha* |
|---|---|---|---|---|---|---|---|
| 25600 | 100 | 34200 | 100 | 22410 | 98 | 3,7 | 7 |
| 12 800 | 100 | 17100 | 100 | 11 205 | 99 | 0,74 | 11 |
| 6400 | 100 | 8550 | 100 | 5 602,50 | 100 | 0,148 | 25 |
| 3200 | 100 | 4275 | 100 | 2801,25 | 100 | 0,085 | 42 |
| 1 600 | 100 | 2 137,50 | 100 | 1400,63 | 100 | 0,0489 | 58 |
| 800 | 100 | 1 068,75 | 100 | 700,31 | 100 | 0,0281 | 65 |
| 400 | 100 | 534,38 | 100 | 350,16 | 100 | 0,0161 | 85 |
| 200 | 100 | 267,19 | 100 | 175,08 | 100 | 0,00925 | 90 |
| 100 | 100 | 133,59 | 100 | 87,54 | 100 | 0,00231 | 95 |
| 50 | 100 | 66,80 | 100 | 43,77 | 100 | 0,000578 | 93 |
| *percentages shown for hTNFα correspond to the mean of values obtained in the three assays. | | | | | | | |

When the product is stored at 4°C in the conditions of Test A, more than 80% of L929 cells are viable, which means that the products show less than 20% of cytolytic activity.

EC50 were calculated for each product and were more than 100 000.

Inactivation Factors were calculated for each product and determined as more than 100 000.

Test B was performed according to Example 4.

The following results are expressed in concentration of total proteins, as determined by the BCA protein assay.

Results are shown in **Figure 13B** and Table 14.

Results show that after 6 weeks at 37°C, the products remain inactivated as more than 50% of L929 cells were viable at a concentration of less than 1000 ng/ml, which means that the product show less than 50% of cytolytic activity.

**Table 14: percentage of cell viability (test B)**

| Conc (ng/ml) | 808 | Conc (ng/ml) | 901 | Conc (ng/ml) | 903 | Conc (ng/ml) | hTNFalpha* |
|---|---|---|---|---|---|---|---|
| 25600 | 16 | 34200 | 11 | 22410 | 22 | 3,7 | 8 |
| 8 533,33 | 24 | 11 400 | 22 | 7470 | 35 | 0,74 | 11 |
| 2 844,44 | 47 | 3800 | 49 | 2490 | 58 | 0,148 | 26 |
| 948,15 | 75 | 1 266,67 | 72 | 830 | 76 | 0,085 | 44 |
| 316,05 | 89 | 422,22 | 93 | 276,67 | 88 | 0,0489 | 62 |
| | | | | | | 0,0281 | 78 |
| | | | | | | 0,0161 | 89 |
| | | | | | | 0,00925 | 93 |
| | | | | | | 0,00231 | 96 |
| | | | | | | 0,000578 | 98 |
| *percentages shown for hTNF correspond to the mean of values obtained in the three assays. | | | | | | | |

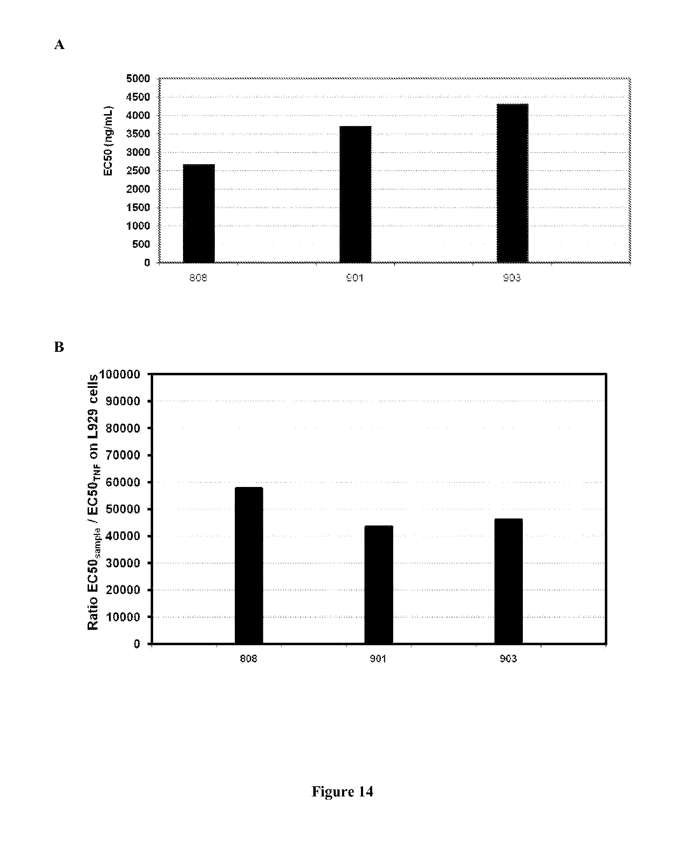

**Figure 14** and Tables 15 and 16 show the EC50 and the Inactivation Factor calculated for each product.

When stored at 37°C for 6 weeks, the products present an EC50 of more than 500 and an Inactivation Factor more than 10000.

**Table 15**

|  | 808 | 901 | 903 |
|---|---|---|---|
| **EC50 (ng/ml)** | 2668 | 3705 | 4310 |

**Table 16**

|  | 808 | 901 | 903 |
|---|---|---|---|
| **EC50$_{sample}$ EC50$_{TNF}$** | 57728 | 43543 | 46044 |
| EC50$_{TNF}$ were calculated using the intra-assay TNF values | | | |

**Claims**

1. An immunogenic product comprising TNF$\alpha$ coupled with KLH, wherein the TNF$\alpha$ is strongly inactivated, which means that the product shows less than 30% of cytolytic activity and/or an inactivation factor of more than 15000, in the conditions of TEST A.

2. The immunogenic product according to Claim **1,** wherein said product remains inactivated overtime, which means that the product shows less than 80% of cytolytic activity and/or an inactivation factor of more than 500, in the conditions of TEST B.

3. The immunogenic product according to Claim **1** or Claim **2,** wherein said product may comprise free TNF$\alpha$ homopolymers of more than 300kDa and when said product comprises free TNF$\alpha$ homopolymers of more than 300kDa, the percentage of free TNF$\alpha$ homopolymers of more than 300kDa is of less than 30% w/w of total TNF$\alpha$ as calculated in TEST C.

4. The immunogenic product according to anyone of Claims **1** to **3,** wherein said product is lyophilized.

5. An immunogenic emulsion comprising a product according to anyone of Claims **1** to **4 ;** and an oil and a surfactant or a mixture thereof; wherein the emulsion is a water-in-oil emulsion or an oil-in-water emulsion, and wherein the oil, the surfactant and/or the mixture of oil and surfactant are pharmaceutically acceptable excipients.

6. The immunogenic emulsion according to Claim **5,** comprising a mixture of oil and surfactant which is an adjuvant, preferably ISA 51.

7. A vaccine composition comprising an immunogenic product according to Claim **1** to **4,** or an emulsion according to anyone of Claims **5** or **6.**

8. A method for preparing a product comprising TNF$\alpha$ coupled with KLH, wherein the TNF$\alpha$ is strongly inactivated, which means that the product shows less than 30% of cytolytic activity in the conditions of TEST A, comprising the steps of:

   a) mixing together (i) purified TNF$\alpha$, (ii) purified Keyhole limpet hemocyanin and (iii) glutaraldehyde
   b) removing compounds having a molecular weight of less than 10 kDa

   **characterized in that** after step b) the following steps are performed:

   c) adding formaldehyde in a concentration/time of reaction condition ranging from at least 60 mM for at least 240 hours to at least 120 mM for at least 144 hours
   d) blocking the reaction with formaldehyde by adding a quenching compound selected from (i) a reducing agent and (ii) an amino acid selected from the group consisting of lysine and glycine and mixture thereof,
   e) collecting the said immunogenic product.

9. The method according to Claim **8,** wherein in step a) glutaraldehyde is applied in a concentration of 1 to 50 mM for more than 110 to less than 400 minutes, preferably 25 mM for 240 minutes.

10. The method according to Claim **8** or Claim **9,** wherein in step c) formaldehyde is applied in a concentration of at least 200 mM during at least 240 hours, preferably of 220 to 270 mM for at least 300 hours.

11. The method according to anyone of Claims **8** to **10,** wherein the reaction with glutaraldehyde is stopped by adding a quenching compound, preferably a quenching compound that is selected from (i) a reducing agent and (ii) an amino acid selected from the group consisting of lysine and glycine and mixture thereof.

12. The method according to anyone of Claims **8** to **11,** wherein prior to collecting at step f), the substances having a molecular weight of less than 300 kDa are removed.

13. A method for preparing a product comprising TNF$\alpha$ coupled with KLH, wherein the TNF$\alpha$ is strongly inactivated, which means that the product shows less than 30% of cytolytic activity in the conditions of TEST A, comprising the steps of:

   a) mixing together (i) purified TNF$\alpha$, (ii) purified Keyhole limpet hemocyanin and (iii) glutaraldehyde
   b) removing compounds having a molecular weight of less than 10 kDa **characterized in that**

in step a) glutaraldehyde is applied at a concentration of at least 20mM during more than 18 hours, the reaction with glutaraldehyde is stopped by adding a quenching compound, preferably a quenching compound that is selected from (i) a reducing agent and (ii) an amino acid selected from the group consisting of lysine and glycine and mixture thereof, and then the product is collected.

14. The method according to Claim **13,** wherein after step b) and prior to collecting the product, formaldehyde is applied in a concentration/time of reaction condition ranging from at least 60 mM for at least 4 days, and then the reaction with formaldehyde is blocked by adding a quenching compound selected from (i) a reducing agent and (ii) an amino acid selected from the group consisting of lysine and glycine and mixture thereof.

15. The method according to Claim **13** or Claim **14,** wherein prior to collecting the product, a step of tangential flow filtration using a filtration membrane having a cut-off value of at least 100 kDa. (pref 300 kDa) is performed.

16. A vaccine composition according to Claim 7 for use in preventing or treating a disease linked to an over-production of TNF$\alpha$ comprising a step of administering to the animal, including a human, in need thereof.

17. The vaccine composition according to Claim **16,** wherein the disease linked to an over-production of TNF$\alpha$ is selected from the group consisting of ankylosing spondylitis, psoriasis, rhumatoïd arthritis, Juvenile idiopathic arthritis, Inflammatory Bowel Disease, Crohn's disease, cachexia, and cancer.

18. A kit comprising, at least one vial containing an immunogenic product according to Claim **1** to **4,** at least one vial containing water for injection, and at least one vial containing adjuvant, and means for mixing the immunogenic product and the water in order to obtain an aqueous solution, and for contacting said solution to the adjuvant, and for emulsifying the mixture of the aqueous solution with the adjuvant, said kit further including at least one needle.

19. A medical device comprising an immunogenic product according to Claim **1** to **4,** or an emulsion according to anyone of Claims **5** or **6** or a vaccine composition according to Claim 7.

**Amended claims in accordance with Rule 137(2) EPC.**

1. An immunogenic product comprising TNF$\alpha$ coupled with KLH, wherein the TNF$\alpha$ is strongly inactivated, which means that the product at a concentration of 100 ng/ml shows less than 30% of cytolytic activity and/or an inactivation factor of more than 15000, in the conditions of TEST A.

2. The immunogenic product according to Claim **1**, wherein said product remains inactivated overtime, which means that the product at a concentration of 100 ng /ml shows less than 80% of cytolytic activity and/or an inactivation factor of more than 500, in the conditions of TEST B.

**3.** The immunogenic product according to Claim **1** or Claim **2**, wherein said product may comprise free TNFα homopolymers of more than 300kDa and when said product comprises free TNFα homopolymers of more than 300kDa, the percentage of free TNFα homopolymers of more than 300kDa is of less than 30% w/w of total TNFα as calculated in TEST C.

**4.** The immunogenic product according to anyone of Claims **1** to **3**, wherein said product is lyophilized.

**5.** An immunogenic emulsion comprising a product according to anyone of Claims 1 to 4 ; and an oil and a surfactant or a mixture thereof; wherein the emulsion is a water-in-oil emulsion or an oil-in-water emulsion, and wherein the oil, the surfactant and/or the mixture of oil and surfactant are pharmaceutically acceptable excipients.

**6.** The immunogenic emulsion according to Claim **5**, comprising a mixture of oil and surfactant which is an adjuvant, preferably ISA 51.

**7.** A vaccine composition comprising an immunogenic product according to Claim 1 to 4, or an emulsion according to anyone of Claims **5** or **6**.

**8.** A method for preparing a product comprising TNFα coupled with KLH, wherein the TNFα is strongly inactivated, which means that the product at a concentration of 100 ng/ml shows less than 30% of cytolytic activity in the conditions of TEST A, comprising the steps of:

a) mixing together (i) purified TNFα, (ii) purified Keyhole limpet hemocyanin and (iii) glutaraldehyde
b) removing compounds having a molecular weight of less than 10 kDa **characterized in that** after step b) the following steps are performed:
c) adding formaldehyde in a concentration/time of reaction condition ranging from at least 60 mM for at least 240 hours to at least 120 mM for at least 144 hours,
d) blocking the reaction with formaldehyde by adding a quenching compound selected from (i) a reducing agent and (ii) an amino acid selected from the group consisting of lysine and glycine and mixture thereof,
e) collecting the said immunogenic product.

**9.** The method according to Claim 8, wherein in step a) glutaraldehyde is applied in a concentration of 1 to 50 mM for more than 110 to less than 400 minutes, preferably 25 mM for 240 minutes.

**10.** The method according to Claim 8 or Claim 9, wherein in step c) formaldehyde is applied in a concentration of at least 200 mM during at least 240 hours, preferably of 220 to 270 mM for at least 300 hours.

**11.** The method according to anyone of Claims 8 to 10, wherein the reaction with glutaraldehyde is stopped by adding a quenching compound, preferably a quenching compound that is selected from (i) a reducing agent and (ii) an amino acid selected from the group consisting of lysine and glycine and mixture thereof.

**12.** The method according to anyone of Claims 8 to 11, wherein prior to collecting at step f), the substances having a molecular weight of less than 300 kDa are removed.

**13.** A vaccine composition according to Claim 7 for use in preventing or treating a disease linked to an over-production of TNFα comprising a step of administering to the animal, including a human, in need thereof.

**14.** The vaccine composition according to Claim 13, wherein the disease linked to an over-production of TNFα is selected from the group consisting of ankylosing spondylitis, psoriasis, rhumatoid arthritis, Juvenile idiopathic arthritis, Inflammatory Bowel Disease, Crohn's disease, cachexia, and cancer.

**15.** A kit comprising, at least one vial containing an immunogenic product according to Claim **1** to **4**, at least one vial containing water for injection, and at least one vial containing adjuvant, and means for mixing the immunogenic product and the water in order to obtain an aqueous solution, and for contacting said solution to the adjuvant, and for emulsifying the mixture of the aqueous solution with the adjuvant, said kit further including at least one needle.

**16.** A medical device comprising an immunogenic product according to Claim **1** to 4, or an emulsion according to anyone of Claims **5** or **6** or a vaccine composition according to Claim 7.

A

B

Figure 1

A

B

Figure 2

A

B

Figure 3

**A**

**B**

### Inactivation factor

Figure 4

Figure 5

Figure 6

A.

B.

Figure 7

A

B

**Figure 8**

## Clinical assessment of arthritis in huTNFα transgenic mice

Figure 9

**Figure 10**

Figure 11

Figure 12

A

B

Figure 13

A

B

Figure 14

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/022813 A2 (NEOVACS [FR]; ZAGURY DANIEL [FR]; LE BUANEC HELENE [FR]) 1 March 2007 (2007-03-01) * the whole document * | 1-19 | INV. A61K39/39 A61K47/48 C07K14/525 |
| X | LE BUANEC HELENE ET AL: "TNF alpha kinoid vaccination-induced neutralizing antibodies to TNF alpha protect mice from autologous TNF alpha-driven chronic and acute inflammation", PROCEEDINGS OF THE NATIONAL ACADEMY OF SCIENCES OF THE UNITED STATES (PNAS), NATIONAL ACADEMY OF SCIENCE, US, vol. 103, no. 51, 19 December 2006 (2006-12-19), pages 19442-19447, XP009147121, ISSN: 0027-8424 * the whole document * | 1-19 | |
| A | US 6 093 405 A (ZAGURY DANIEL [FR] ET AL) 25 July 2000 (2000-07-25) * the whole document * | 1-19 | TECHNICAL FIELDS SEARCHED (IPC) A61K C07K |
| A | DELAVALLEE LAURE ET AL: "Active immunization to tumor necrosis factor-alpha is effective in treating chronic established inflammatory disease: a long-term study in a transgenic model of arthritis", ARTHRITIS RESEARCH AND THERAPY, BIOMED CENTRAL, LONDON, GB, vol. 11, no. 6, 23 December 2009 (2009-12-23), page R195, XP021070720, ISSN: 1478-6354 * the whole document * | 1-19 | |

-/--

The present search report has been drawn up for all claims

| Place of search | Date of completion of the search | Examiner |
|---|---|---|
| Munich | 29 April 2011 | Morawetz, Renate |

EPO FORM 1503 03.82 (P04C01)

EP 2 462 950 A1

Europäisches
Patentamt
European
Patent Office
Office européen
des brevets

**EUROPEAN SEARCH REPORT**

Application Number

EP 10 19 4240

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BIZZINI B ET AL: "KINOIDS: THE BASIS FOR ANTICYTOKINE IMMUNIZATION AND THEIR USE IN HIV INFECTION", MOLECULAR CELL BIOLOGY, XX, XX, vol. 41, no. 3, 1 May 1995 (1995-05-01), pages 351-356, XP008060572, * the whole document * | 1-19 | |

-----

TECHNICAL FIELDS
SEARCHED      (IPC)

The present search report has been drawn up for all claims

| Place of search | Date of completion of the search | Examiner |
|---|---|---|
| Munich | 29 April 2011 | Morawetz, Renate |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.** EP 10 19 4240

This annex lists the patent family members relating to the patent documents cited in the above-mentioned European search report.
The members are as contained in the European Patent Office EDP file on
The European Patent Office is in no way liable for these particulars which are merely given for the purpose of information.

29-04-2011

| Patent document cited in search report | | Publication date | Patent family member(s) | | | Publication date |
|---|---|---|---|---|---|---|
| WO 2007022813 | A2 | 01-03-2007 | AT | 500848 | T | 15-03-2011 |
| | | | AU | 2006284248 | A1 | 01-03-2007 |
| | | | CA | 2609572 | A1 | 01-03-2007 |
| | | | CN | 101222941 | A | 16-07-2008 |
| | | | EP | 1888122 | A2 | 20-02-2008 |
| | | | JP | 2008542227 | T | 27-11-2008 |
| | | | NZ | 563526 | A | 28-02-2009 |
| | | | US | 2008193473 | A1 | 14-08-2008 |
| | | | ZA | 200710011 | A | 29-10-2008 |
| US 6093405 | A | 25-07-2000 | AT | 185149 | T | 15-10-1999 |
| | | | AU | 2147992 | A | 12-01-1993 |
| | | | CA | 2111580 | A1 | 23-12-1992 |
| | | | DE | 69230068 | D1 | 04-11-1999 |
| | | | DE | 69230068 | T2 | 09-03-2000 |
| | | | DK | 0591281 | T3 | 20-12-1999 |
| | | | EP | 0591281 | A1 | 13-04-1994 |
| | | | ES | 2136088 | T3 | 16-11-1999 |
| | | | FR | 2677654 | A1 | 18-12-1992 |
| | | | WO | 9222577 | A1 | 23-12-1992 |
| | | | GR | 3032037 | T3 | 31-03-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9846642 A
- WO 0211759 A
- WO 2004024189 A
- WO 2007022813 A

- WO 200424189 A
- EP 109942 A
- EP 180564 A
- EP 231039 A
- GB 2189141 A
- US 4877612 A

**Non-patent literature cited in the description**

- **MCGHEE, J. R. et al.** On vaccine development. *Sem. Hematol.,* 1993, vol. 30 (3-15), 5
- **BOVARNIK et al.** *J. Bacteriology,* 1950, vol. 59, 509
- **LEHMANN et al.** *JEM,* 1987, vol. 165, 657-663
- **HAYWARD et al.** *BMC Physiology,* 2007, vol. 7, 13-29
- **LEE et al.** *J Pharmacol Sci,* 2009, vol. 109, 211-221

- **NABER et al.** *The Journal of Medicine,* vol. 61 (4), 105-110
- **TARGAN.** *NEJM,* 1997, vol. 340, 1029-35
- *Gastroenterology,* 2004, vol. 126, 402
- **HANAUER.** *Gastroenterology,* 2006, vol. 130 (6), 1929-30
- **COLOMBE.** *Am Journ Gastroenterol.,* 2007, vol. 102 (2), 5496-7
- **SCHREIBER.** *NEJM,* 2007, vol. 357 (13), 1357